Europäisches Patentamt

European Patent Office

Office européen des brevets

(19)

(11) **EP 1 026 616 A1**

(12) **EUROPÄISCHE PATENTANMELDUNG**

(43) Veröffentlichungstag:
09.08.2000 Patentblatt 2000/32

(51) Int Cl.$^7$: **G06F 19/00**, G06F 17/18

(21) Anmeldenummer: **99105884.3**

(22) Anmeldetag: **24.03.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **05.02.1999 EP 99102275**

(71) Anmelder: **Liebel, Franz-Peter, Dr.**
**D-33619 Bielefeld (DE)**

(72) Erfinder: **Liebel, Franz-Peter, Dr.**
**D-33619 Bielefeld (DE)**

(54) **Verfahren zur Ermittlung von Wahrscheinlichkeiten pathophysiologischer Zustände als Zwischenergebnisse der Diagnosefindung**

(57)    Es wird ein Verfahren vorgestellt, das für eine vorgegebene Symptommenge die Zutreffenswahrscheinlichkeiten der kausal zugeordneten Ereignisse zu berechnen vermag. Dies entspricht - im Fall einer Anwendung in der Medizin - der Berechnung von Wahrscheinlichkeiten pathophysiologischer Zustände.

Für die Berechnungen werden Ereignisse mit sicher bestimmter, sowie Ereignisse mit unbekannter Zutreffenswahrscheinlichkeit eingesetzt. Für jede der unbekannten Zutreffenswahrscheinlichkeiten stellt man eine Bestimmungsgleichung auf und erhält so bei x Unbekannten ein System von x nicht-linearen Gleichungen.

Mit der Einführung von Voraussetzungen, die im hauptsächlich vorgesehenen Anwendungsbereich der medizinischen Diagnostik gut zu erfüllen sind, können die in den Gleichungen erscheinenden bedingten Wahrscheinlichkeiten so vereinfacht werden, daß die jeweiligen Bedingungen im allgemeinen nur noch ein Ereignis aufweisen. Dadurch wird die Erstellung von Stichproben zur Ermittlung der entsprechenden Zahlenwerte problemlos.

EP 1 026 616 A1

**Beschreibung**

A ) Verwendbarkeit der Erfindung

Es ist problematisch, die im Verlauf eines medizinischen Diagnoseprozesses angehäuften Daten und Meßwerte zu überblicken und zu werten. Eine entscheidende Hilfe kann ein Verfahren geben, das für die angefallenem Meßwerte kausal zugeordnte pathophysiologische Zustände bestimmt, indem es deren Zutreffenswahrscheinlichkeiten berechnet.

Ein solches Verfahren ist dann vergleichbar einem Zusammenfassen und Bündeln einzelner Daten zur Gewinnung einer Meßgröße oder einer Aussage für einen "übergeordneten" pathologischen Zustands des Gewebes.

Im Hinblick auf die oft nur vage zielgerichteten Laboruntersuchungen, und im Hinblick auf die Notwendigkeit gezielt vorgenommener Untersuchungen mit bildgebenden medizinischen Geräten, kann das vorgestellte Verfahren integraler Bestandteil werden von z.B. Laborautomaten, Computertomographen u.ä.

B) Stand der Technik und Vorteile des Verfahrens

Zu Anspruch 1

Durch die in Anspruch 1 angegebene Gleichung vermeidet man a-priori-Wahrscheinlichkeiten (z.B. p(H), p($K_1$),... ), deren Zahlenwerte nur schwer zu ermitteln sind.

Für eine Wahrscheinlichkeit der Form p(F | K J H) ist es wesentlich, daß die Operationen "Interpolation" und "Zerlegung bzgl. der Ereignisse vor dem Bedingungsstrich" in genau dieser Reihenfolge ausgeführt werden.

Zu Anspruch 2

Da die im Bedingungsverbund einer bedingten Wahrscheinlichkeit stehenden '-gekennzeichneten Ereignisse Separationseigenschaft besitzen ( vergleiche Abschnitt C), Erläuterung zum Linearen Interpolationssatz ), ist eine lineare Interpolation nur auszuführen, wenn die Voraussetzung gem. Anspruch 2 erfüllt ist.

Zu Anspruch 3

Es hat sich gezeigt, daß bei nicht-linearer Interpolation eine Gleichbehandlung aller '-gekennzeichneten Ereignisse die nötige Einfachheit herbeiführt.

Interpolationen mit verschiedenen $V_{E'_i}$, oder gar unstetige Interpolationen, sind aufgrund des beträchtlichen Aufwands nicht geeignet.

Interpoliert man gleichbleibend mit maximalem $V_{E'_i}$, so ist man, bei Berücksichtigung der Ereignisse des Bedingungsverbundes, nächstmöglich an der linearen Interpolation.

Zu Anspruch 4 und 5

Mit einer einzigen "wirklichen" Voraussetzung, nämlich der Unabhängigkeit der Ereignisse in

$$\bigcup_i (URS(F_i) \cup INH(F_i)),$$

erreicht man die Lösung der Aufgabe.

Die Kritik, dadurch sei das Postulat der Unabhängigkeit lediglich von der Folgen-Ebene auf die Hypothesen-Ebene verlagert worden, trifft nicht zu, da die Folgen wegen gemeinsamer Ursachen per definitionem abhängig sind; eine solche Abhängigkeit liegt für die Ereignisse der H/K-Ebene i.a. nicht vor.

Zu Anspruch 6

Es gelingt die Zerlegung von bedingten Wahrscheinlichkeiten, auch wenn sowohl vor als auch nach dem Bedingungsstrich ein unter Umständen umfangreicher Ereignisverbund steht.

Man erreicht durch die Verkleinerungen schließlich bedingte Wahrscheinlichkeiten, die vor und nach dem Bedingungsstrich nur noch ein einzelnes Ereignis aufweisen. Da die Wahrscheinlichkeiten zudem von der Form *p (Folge|Ursache)* sind, wird die Ermittlung der zugehörigen Zahlenwerte aus Stichproben problemlos.

Zu Anspruch 7

Durch die gleichzeitige Erfassung aller unbekannten Zutreffenswahrscheinlichkeiten in einem Gleichungssystem ist die Schwierigkeit bewältigt, daß sich die '-gekennzeichneten Ereignisse einer kausalen Struktur wechselseitig beeinflussen.

Zu Anspruch 8 und Anspruch 9

Man kann bei Anspruch 6 so vorgehen, daß man "from the scratch" für jede zu verkleinernde Wahrscheinlichkeit den A→L-Satz bzw. das A→L-Korollar 1 anwendet, oder aber daß man sozusagen als "ready mix" die Beziehungen des Anspruchs 8 oder 9 nutzt.

Diese Beziehungen ergeben sich eindeutig und unmittelbar aus den Formeln und Gleichungen, sowie den getroffenen Voraussetzungen, wie sie in der Patentanmeldung enthalten sind, auf die sich der Prioritätsanspruch bezieht.

Die Beweisführung hierzu ist in Abschnitt C) enthalten (vergl. die Beweise des Ursachen-Faktorisierungssatzes und des kombinierten Faktorisierungssatzes). Man erreicht Bedingungsverbunde, die nur noch eine einzige unnegierte Ursache enthalten, zusammen mit höchstens einem zugehörigen Inhibitor.

Zu Anspruch 10

Setzt man vollzählig vorliegende Ursachen voraus, so ist auch zum Beispiel $p(F_1|\bar{H}\cdot\bar{K}_1...\bar{K}_n) = 0$ vorausgesetzt. Trifft dies in der Praxis jedoch nicht zu, d.h. ist z.B. $p(F_1|\bar{H}\cdot\bar{K}_1...\bar{K}_n) > 0$, so sind Korrekturen gem. Anspruch 10 angebracht. Diese Korrekturfaktoren stellen zugleich die Fehler dar, die man dann begeht, wenn man die Verfahren der Ansprüche 8 und 9 einsetzt, ohne die Existenz unbekannter Ursachen zu berücksichtigen.

Zur Problematik unabhängiger und abhängiger Patentansprüche

Gelingt es,

$$p(H|U\,F\,K\,I\,J) = \cfrac{1}{1+\cfrac{p(F|KJ\bar{H})p(\bar{H}|UI)}{p(F|KJH)p(H|UI)}}$$

durch geeignete Voraussetzungen zu erreichen, so ist man, falls die verwendeten Verbunde extrem wenige und sicher bestimmte Ereignisse enthalten, bereits am Ziel. Selbstverständlich ist dies in der Praxis kaum der Fall, d.h. die Verbunde enthalten im allgemeinen zahlreiche Ereignisse. Die dann unter Umständen umfangreichen Wahrscheinlichkeiten müssen faktorisiert werden, um repräsentative und einfache Stichproben zur Ermittlung der Zahlenwerte zu ermöglichen. Faktorisierungen, der Gegenstand der Ansprüche 6, 8 und 9, sind damit in die Kategorie Sonderaufgaben einzustufen.

Es ist zugelassen, daß (U F K I J) nur ungestrichene Ereignisse enthält. Somit zählen auch die Interpolationen der Ansprüche 2 und 3 zu den Sonderaufgaben, anzuwenden in den besonderen Fällen des Vorliegens von '-Ereignissen. Es werden jedoch bei sinnvollen Anwendungen '-gekennzeichnete Ereignisse häufig zu verarbeiten sein.

Art und Anzahl der Voraussetzungen, um die angegebene Umformung des Anspruchs 1 zu erreichen, sind in Anspruch 1 noch offen. In den Ansprüchen 4 und 5 werden dann schließlich spezielle Voraussetzungen formuliert, die das Gewünschte leisten. Theoretisch kann man, in besonders einfach gelagerten Fällen, auch ohne Anspruch 4 oder 5 auskommen.

Mit Anspruch 7 wird erreicht, daß zusammen mit der Starthypothese H auch die mit H kausal verknüpften Ereignisse unbekannter Zutreffenswahrscheinlichkeit mitverarbeitet werden. Solche Ereignisse bleiben bei herkömmlichen Systemen, da man ihre Zutreffenswahrscheinlichkeit ja nicht kennt, im allgemeinen unberücksichtigt.

Die Ansprüche 8 und 9 ergeben sich aus Anspruch 6; sie stellen eine Vereinfachung dar. Der dann unter Umständen verursachte Fehler wird durch die Korrekturen des Anspruchs 10, die sich ebenso aus dem A→L-Korollar 1 herleiten, wieder ausgeglichen.

C) Darstellung der Lösung und Beispiel

1. Einleitung

Es hat sich als schwierig erwiesen hat, eine brauchbare sogenannte Bewertungsfunktion zu konzipieren. Eine solche Bewertungsfunktion ist ein Verfahren, das anhand einer vorgegebenen Ereignismenge die Wahrscheinlichkeiten kausal verknüpfter Ereignisse, insbesondere von Ursachen ermittelt. Dabei meint "Ermittlung einer unbekannten Wahrscheinlichkeit" die konkrete Berechnung eines Zahlenwertes mit Methoden der Diskreten Stochastik.

Es hat sich zudem gezeigt, daß das kleine Gebiet der Diskreten Stochastik für ein derartiges Unterfangen nicht die benötigten Werkzeuge bereithält. Einen ersten Schritt zur Fortsetzung der klassischen Stochastik durch Einbeziehung unsicher bestimmter Ereignisse liefert die lineare Interpolation

$$p(H|E') = p(H|E)\ p(E|E') + p(H|\bar{E})p(\bar{E}|E'). \qquad (1.1)$$

Im weiteren wird sich recht eindrucksvoll ergeben, daß die Interpolation der Glg. 1.1 ein Sonderfall des Allgemeinen Interpolationssatzes ist, wenn dort gilt: $(E_1...E_h E'_{h+1}...E'_k) := E'$ (vergl. Abschnitt 3., Glg.3.1).

2. Kausalstruktur

Die Ereignisse werden zweckmäßigerweise in einem Kausalnetz geordnet, dem die Bezeichnung L-Netz gegeben wurde. Diese L-Netz ist im wesentlichen dadurch charakterisiert, daß die Ereignisse durch zunächst beliebig viele gerichtete Kausalverweise verknüpft sind, wobei zugelassen ist, daß auf die Kausalverbindungen Inhibitoren einwirken, und daß die Inhibitionsmechanismen wiederum durch hemmende Ereignisse verstärkt oder gemindert werden können.

Eine inhibitorische Beeinflussung einer beliebigen Kausalverbindung $U_3 \to H$ im nachfolgenden Beispiel der Abb.2.1 bedeutet, daß Ereignisse bestehen, die eine Erzeugung von H durch $U_3$ hemmend beeinflussen. Ist $U_3 \to H$ die Kurzbezeichnung des kausalen Vorgangs "$U_3$ erzeugt H", so wird der hemmende Einfluß ausgedrückt durch:

$$p(U_3 \to H|U_3) \geq p(U_3 \to H|U_3\ I_1). \qquad (2.1)$$

Die so gebildete Grundstruktur erhält schließlich durch die Voraussetzungen des Abschnitts 4. die endgültige Gestalt.

Das nachfolgende Beispiel eines L-Netzes enthält negierte und unnegierte Ereignisse, sowie durch □? gekennzeichnete Netzknoten mit unbekannter Zutreffenswahrscheinlichkeit.

Ursachen-Ebene  $U_1$ □  $U_2$ □?  $U_3$ □?  $I_1$ □  $I_2$ □

Hypothesen-Ebene  $K_1$ □?  $J_1$ □  $H$ □?  $K_2$ □  $K_3$ □?  $\overline{J_2}$ □

Folgen-Ebene  $F_1$ □  $F_2$ □  $F_3$ □  $\overline{F}_4$ □  $F_5$ □

Abb.2.1:

Beispiel eines L-Netzes. Die gewählten Bezeichnungen der Netzknoten sind willkürlich. Das Netz kann im Bedarfsfall über die □? – Ereignisse beliebig fortgesetzt werden.

Es werden folgende Bezeichnungen festgelegt, die für den beliebig gewählten Netzknoten H formuliert sind:

URS(H)   Menge der unmittelbaren Ursachen von H.
FOL(H)   Menge der unmittelbaren Folgen von H.
DIFF(H)   Menge der von H verschiedenen Netzknoten, die unmittelbare Ursache für Elemente aus FOL(H) sind. (DIFF(H) umfaßt diejenigen Elemente, die z.B. bei Anwendungen in der Medizin differentialdiagnostisch zu H in Betracht zu ziehen sind.)
INH(Z)   Menge der Netzknoten, die über Schaltstellen die auf einen beliebigen Netzknoten Z hinführenden Kausalverbindungen inhibitorisch beeinflussen.
WERT(H)   Menge aller Netzknoten mit Einfluß auf die Zutreffenswahrscheinlichkeit von H (Wertungsumgebung von H).

Der Menge WERT(H) wird zugewiesen:

$$WERT(H) := URS(H) \cup FOL(H) \cup DIFF(H) \cup INH(H) \cup \bigcup_{Z \in FOL(H)} INH(Z) \, . \ (2.2)$$

Der aus den Elementen der Menge WERT(H) gebildete Verbund (synonym: Produkt, Durchschnitt) wird mit W(H) bezeichnet. Die Ereignisse darin sind dann je nach Zutreffenswahrscheinlichkeit negiert, unnegiert oder '-gekennzeichnet. Mit W(H) wird definiert:

*Definition ('-Kennung)*

*Für die zugrundegelegte Kausalstruktur und ein darin enthaltenes, beliebiges Element H ist H' : = W(H).*
Für das Beispiel der Abb.2.1 erhält man so als Zutreffenswahrscheinlichkeit des Ereignisses H :

$$p(H \mid H') = p(H \mid W(H))$$

$$= p(H \mid U_1\ U_2'\ U_3'\ F_1\ F_2\ F_3\ \overline{F}_4\ F_5\ K_1'\ K_2\ K_3'\ I_1\ I_2\ J_1\ \overline{J}_2). \qquad (2.3)$$

### 3. Interpolationsformeln

Benötigt werden Formeln zur Berechnung von Wahrscheinlichkeiten der Form $p(H \mid E_1...E_hE'_{h+1}...E'_k)$, wobei die Ereignisse $E_1$, ..., $E_h$ sicher bestimmt, d.h. negiert oder unnegiert vorliegen, während die Ereignisse $E_i'$, i = h+1, ..., k, eine (bekannte oder unbekannte) Zutreffenswahrscheinlichkeit $0 < p(E_i \mid E_i') < 1$ besitzen und demzufolge eine '-Kennung tragen.

In den Formeln werden Ausdrücke der Form $p(E_i \mid V_{E'_i})$, i = h + 1, ..., k verwendet, wobei $V_{E'_i}$ einen auch leer zugelassenen Verbund bezeichnet, der aus dem Verbund $(E_1...E_hE'_{h+1}...E'_k)$ dadurch entsteht, daß man $E'_i$ entfernt, sowie beliebige und beliebig viele weitere Elemente.

Für die Interpolationsfunktionen zur Berechnung von $p(H \mid E_1...E_hE'_{h+1}...E'_k)$ wird gefordert, daß die Interpolation für alle i = h+1 ... , k die nachfolgenden drei Interpolationspunkte erfüllt:

### 1. Interpolationspunkt:

$$p(H|E_1...E_hE'_{h+1}...E'_i...E'_k) = p(H|E_1...E_hE'_{h+1}...\bar{E}_i...E'_k) \text{ für } p(E_i|E'_i) = 0.$$

### 2. Interpolationspunkt:

$$p(H|E_1...E_hE'_{h+1}...E'_i...E'_k) = p(H|E_1...E_hE'_{h+1}...E_i...E'_k) \text{ für } p(E_i|E'_i) = 1.$$

### 3. Interpolationspunkt:

$$p(H|E_1...E_hE'_{h+1}...E'_i...E'_k) = p(H|E_1...E_hE'_{h+1}...E'_{i-1}E'_{i+1}...E'_k)$$

für

$$p(E_i \mid E'_i) = p(E_i \mid V_{E'_i})$$

bei beliebig gewähltem $V_{E'_{h+1}}$.

(Die Bedeutung des 3. Interpolationspunktes besteht darin, daß genau dann, wenn $p(E_i|E'_i)$ den Wert $p(E_i|V_{E'_i})$ besitzt, kein updating von p(H | H') bzgl. $E'_i$ stattfindet.)

*Allgemeiner Interpolationssatz*

*Für $p(H \mid E_1...E_hE'_{h+1}...E'_k)$ gelte:*

- *$E_1$, ..., $E_h$ sind negiert oder unnegiert (beliebig aber fest).*
- *$E'_{h+1},....,E'_k$ besitzen $0 < p(E_i \mid E'_i) < 1$, i = h+1, ... , k.*
- *Es sei $q_i \in \{0,1\}$, i = h + 1, ..., + k mit $E_i^{(q_i)} = \bar{E}_i$ für $q_i = 0$ und $E_i^{(qi)} = E_i$ für $q_i = 1$.*
- *$V_{E'_i}$ ist ein beliebiger, auch leer zugelassener Teilverbund von*

$$(E_1...E_h E'_{h+1}...E'_{i-1}E'_{i+1}...E'_k), \; i = h+1, ..., k.$$

*Dann genügen folgende Gleichungen den geforderten drei Interpolationspunkten:*

$$p(H \mid E_1...E_h E'_{h+1}...E'_k) = \frac{\displaystyle\sum_{q_{h+1},...,q_k=0,1} p(HE_1...E_h E_{h+1}^{(q_{h+1})}...E_k^{(q_k)}) \prod_{i=h+1}^{k} \frac{p(E_i^{'(q_i)} \mid E'_i)}{p(E_i^{(q_i)} \mid V_{E_i})}}{\displaystyle\sum_{q_{h+1},...,q_k=0,1} p(E_1...E_h E_{h+1}^{(q_{h+1})}...E_k^{(q_k)}) \prod_{i=h+1}^{k} \frac{p(E_i^{'(q_i)} \mid E'_i)}{p(E_i^{(q_i)} \mid V_{E_i})}} \; , \; (3.1)$$

$$E_i^{'(0)} = \overline{E_i} \; und \; E_i^{'(1)} = E_i, \; i = h+1, ..., k.$$

Beweis: Elementar.

Die Anwendung der Glg.3.1 oder eines davon abgeleiteten Satzes ist ein updating-Verfahren.

Das updating so gewählt, daß p(H | H') infolge $E_i$' genau dann nicht verändert wird, wenn p($E_i$ | $E_i$') den Wert besitzt, den es durch das Vorliegen der ohnehin für p(H|H') verwendeten Ereignisse ($E_1...E_h E'_{h+1}...E'_{i-1}E'_{i+1}...E'_k$) erreichen kann, wenn also $p(E_i|E'_i) = P(E_i|E_1...E_h E'_{h+1}...E'_{i-1}E'_{i+1}...E'_k)$ gilt.

Für das updating im L-Netz wird somit i.a. das maximale $V_{E'_i}$ eingesetzt, d.h. es wird $V_{E'_i} := (E_1...E_h E'_{h+1}...E'_{i-1}E'_{i+1}...E'_k)$ verwendet.

Am Beispiel p($U_2$|$U_1$ $I_1$ $I_2$ H' $U_3$') wird die Handhabung der Glg.3.1 demonstriert. Hierzu werden $V_{H'}$ und $V_{U'_3}$ größtmöglich gewählt zu $V_{H'} := (U_1 I_1 I_2 U'_3)$ bzw. $V_{U'_3} := (U_1 I_1 I_2 H')$. (Hier wäre jeweils auch jeder Teilverbund möglich, einschließlich des leeren Verbunds.)

(Wählt man etwa $V_{U'_3} := (U_1 I_1 I_2)$, so erscheint wegen p($U_3$|$U_1$ $I_1$ $I_2$) = p($U_3$) eine der a-priori-Wahrscheinlichkeiten, deren Zahlenwerte schwer zu ermitteln sind.) Es ergibt sich gem. Glg.3. 1:

$$p(U_2 \mid U_1\, I_1\, I_2\, H^{\prime}\, U_3{}^{\prime}) \qquad\qquad (3.2)$$

$$= \cfrac{p(U_2 U_1 I_1 I_2 H U_3)\cfrac{p(H\mid H')}{p(H\mid U_1 I_1 I_2 U_3')}\cfrac{p(U_3\mid U_3')}{p(U_3\mid U_1 I_1 I_2 H')} +}{p(U_1 I_1 I_2 H U_3)\cfrac{p(H\mid H')}{p(H\mid U_1 I_1 I_2 U_3')}\cfrac{p(U_3\mid U_3')}{p(U_3\mid U_1 I_1 I_2 H')} +} \;\ldots$$

$$\ldots\; \cfrac{p(U_2 U_1 I_1 I_2 H \overline{U_3})\cfrac{p(H\mid H')}{p(H\mid U_1 I_1 I_2 U_3')}\cfrac{p(\overline{U_3}\mid U_3')}{p(\overline{U_3}\mid U_1 I_1 I_2 H')} +}{p(U_1 I_1 I_2 H \overline{U_3})\cfrac{p(H\mid H')}{p(H\mid U_1 I_1 I_2 U_3')}\cfrac{p(\overline{U_3}\mid U_3')}{p(\overline{U_3}\mid U_1 I_1 I_2 H')} +} \;\ldots$$

$$\ldots\; \cfrac{p(U_2 U_1 I_1 I_2 \overline{H} U_3)\cfrac{p(\overline{H}\mid H')}{p(\overline{H}\mid U_1 I_1 I_2 U_3')}\cfrac{p(U_3\mid U_3')}{p(U_3\mid U_1 I_1 I_2 H')} +}{p(U_1 I_1 I_2 \overline{H} U_3)\cfrac{p(\overline{H}\mid H')}{p(\overline{H}\mid U_1 I_1 I_2 U_3')}\cfrac{p(U_3\mid U_3')}{p(U_3\mid U_1 I_1 I_2 H')} +} \;\ldots$$

$$\ldots\; \cfrac{p(U_2 U_1 I_1 I_2 \overline{H U_3})\cfrac{p(\overline{H}\mid H')}{p(\overline{H}\mid U_1 I_1 I_2 U_3')}\cfrac{p(\overline{U_3}\mid U_3')}{p(\overline{U_3}\mid U_1 I_1 I_2 H')}}{p(U_1 I_1 I_2 \overline{H U_3})\cfrac{p(\overline{H}\mid H')}{p(\overline{H}\mid U_1 I_1 I_2 U_3')}\cfrac{p(\overline{U_3}\mid U_3')}{p(\overline{U_3}\mid U_1 I_1 I_2 H')}} \;.$$

An dem Beispiel ist auch leicht die Erfüllung der Interpolationspunkte zu sehen.

*Spezielle Interpolationssätze*

*L-Satz*

*Mit $V_{E'_i} := \varnothing$ für alle i = h+1,...,k ergibt sich aus Glg.3.1 ein einfacherer Interpolationssatz, der infolge seiner Entstehungsgeschichte die Bezeichnung L-Satz führt.*

Linearer *Interpolationssatz*

*Mit $V_{E'_i} := (E_1...E_h)$ ergibt sich aus Glg.3.1 die "Lineare Interpolation" (linear in den $p(E_i \mid E'_i)$), falls zusätzlich a) oder b) gilt:*

*a) $(E'_{h+2}...E'_k) = \varnothing$*
*b) $\{E_{h+1},... E_k\}$ sind unter der Bedingung $(E_1.....E_h W(E_{h+1})^*.....W(E_k)^*)$ stochastisch unabhängig, wobei $W(E_i)^* := (W(E_i)$ abzgl. $\{H\} \cup \{E_1,..., E_h\})$.*

*Mit den Voraussetzungen a) oder b) erhalt man aus Glg.3.1:*

$$p(H \mid E_1...E_h E'_{h+1}...E'_k) = \sum_{q_{h+1},...,q_k=0,1} p(H \mid E_1...E_h E^{(q_{h+1})}_{h+1}...E^{(q_k)}_k) \prod_{i=h+1}^{k} p(E^{(q_i)}_i \mid E'_i),$$

$$E^{(0)}_i = \overline{E_i} \ \text{ und } \ E^{(1)}_i = E_i, \ i = h+1, ..., k. \tag{3.3}$$

Erläuterung zu b):

Gegeben sei die Struktur:

$$K_1\,\square? \qquad K_2\,\square?$$

$$F_1\,\square \qquad F_2\,\square$$

(Nur eingezeichnete Ereignisse sind existent.)

Vorbemerkung

Es ist $p(F_1|K_1' K_2') \neq p(F_1|K_1' K_2' F_2) \neq p(F_1|K_1' K_2' \overline{F}_2)$. Jede dieser Wahrscheinlichkeiten kann sich auf eine andere Kausalstruktur, nämlich eine Struktur ohne $F_2$, mit $F_2$ und mit $\overline{F}_2$ beziehen.

Es gilt jedoch $p(F_1|K_1' K_2') = p(F_1|K_1' K_2' F_2)$, wenn vorausgesetzt wird, daß nur eine Kausalstruktur zugrunde liegt. Diese Kausalstruktur ist bestimmend für die $p(K_1|K_1')$ und $p(K_2|K_2')$, die zwar unbekannt, bei vorgegebener Struktur aber fest sind. Bezeichnung: $K_1'$, $K_2'$ _separieren_ $F_1$ von $F_2$.

Für die angegebene Struktur wird nun gezeigt, daß die Anwendung der Glg.3.1 auf $p(F_1|W(F_1))$ keine lineare Interpolation ergibt:

$$p(F_1 \mid W(F_1)) = p(F_1 \mid K_1' K_2')$$

$$= {}_{\text{(Separationseigenschaft)}} \, p(F_1 \mid K_1' K_2' W(K_1)^* \ W(K_2)^*)$$

$$= p(F_1 \mid K_1' K_2' F_2)$$

$$= (\text{Anwendung der Glg.3.1 mit } V_{K_1} = V_{K_2} := (F_2))$$

$$= {}_{\text{(Glg.3.1)}} \frac{p(F_1 F_2 K_1 K_2)\dfrac{p(K_1 \mid K_1')\, p(K_2 \mid K_2')}{p(K_1 \mid F_2)\, p(K_2 \mid F_2)} + .....}{p(F_2 K_1 K_2)\dfrac{p(K_1 \mid K_1')\, p(K_2 \mid K_2')}{p(K_1 \mid F_2)\, p(K_2 \mid F_2)} + .....} \, .....$$

$$\neq \ p(F_1 \mid F_2 K_1 K_2)\, p(K_1 \mid K_1')\, p(K_2 \mid K_2') + .....$$

$$(\text{wegen } p(K_1 K_2 \mid F_2) \neq p(K_1 \mid F_2)\, p(K_2 \mid F_2))$$

$$= {}_{\text{(unabhängig)}} \, p(F_1 \mid K_1 K_2)\, p(K_1 \mid K_1')\, p(K_2 \mid K_2') + .....$$

Wegen Nichterfüllung der Ziff. b) liefert Glg.3.1 somit keine linare Interpolation.

Die Formel (3.3) wird am Beispiel $p(H|U_1 I_1 I_2 U_2' U_3')$ verdeutlicht. Bei stochastischer Unabhängigkeit von $U_2$ und $U_3$ unter der Bedingung $(U_1 I_1 I_2)$, sowie der Abwesenheit ent-separierender Ereignisse in $U_2'$, $U_3'$, ergibt sich:

$p(H \mid U_1 \ I_1 \ I_2 \ U_2' \ U_3') =$
  $p(H \mid U_1 \ I_1 \ I_2 \ U_2 \ U_3)\, p(U_2 \mid U_2')\, p(U_3 \mid U_3') +$
  $p(H \mid U_1 \ I_1 \ I_2 \ U_2 \ \overline{U}_3)\, p(U_2 \mid U_2')\, p(\overline{U}_3 \mid U_3') +$
  $p(H \mid U_1 \ I_1 \ I_2 \ \overline{U}_2 \ U_3)\, p(\overline{U}_2 \mid U_2')\, p(U_3 \mid U_3') +$

$$p(H \mid U_1\ I_1\ I_2\ \overline{U}_2\ \overline{U}_3\ )\ p(\overline{U}_2 \mid U_2')\ p(\overline{U}_3 \mid U_3'). \qquad (3.4)$$

Die Glgn.3.1 und 3.3 sind Interpolationen und im allgemeinen keine Gleichheit. (Kriterien für die Erreichung von Gleichheit vergl. Abschnitt 7.)

4. Voraussetzungen

Die L-Netze werden durch die nachstehenden Voraussetzungen so strukturiert, daß sich die vorgesehenen Berechnungen vereinfachen. Dabei ist zu beachten, daß die zugrunde gelegte Kausalstruktur, d.h. die Verknüpfungen der pathophysiologischen Zustände, solche Voraussetzungen zulassen. Es gelten Bezeichnungen, die für den beliebigen Knoten H am Beispiel der Abb.2.1 formuliert sind:

U   Verbund der Elemente aus $\underline{U}RS(H)$; $U := (U_1\ U_2'\ U_3')$.
F   Verbund der Elemente aus $\underline{F}OL(H)$; $F := (F_1\ F_2\ F_3\ \overline{F}_4\ F_5)$.
K   Verbund der Elemente aus $\overline{D}IFF(H)$; $K := (K_1'\ K_2\ K_3')$. ( K erinnert an "$\underline{k}$onkurrierend zu H".)
I   Verbund der Elemente aus $\underline{I}NH(H)$; $I := (I_1\ I_2)$.
J   Verbund der Elemente aus $\underline{I}NH(F_1),...,\ INH(F_5)$; $J := (J_1\ \overline{J}_2\ )$.

*Die folgenden, für H formulierten Voraussetzungen I, IIa, IIb, IIc und III, sowie die ergänzenden Voraussetzungen IIa\*, IIb\* und IIc\* gelten entsprechend für jeden nicht-inhibitorischen Netzknoten mit unbekannter Zutreffenswahrscheinlich*keit.

*Voraussetzung I*

*Die Ereignisse aus F, 1 und J liegen mit der Wahrscheinlichkeit Null oder Eins vor.*

Erläuterung zu Voraussetzung I

Das gestellte Problem ist die Ermittlung der Wahrscheinlichkeiten hypothetischer Ursachen, also der Wahrscheinlichkeiten für Ereignisse aus der H/K-Ebene, der U-Ebene oder "weiter darüber liegender" Ursachen (vergl. Abb. 2.1). Grundlage für eine solche Ermittlung sind Erkenntnisse über Folge-Ereignisse und Inhibitoren, die zugunsten der Ergebnisgenauigkeit nicht unsicher bestimmt sein sollen.

*Voraussetzung IIa*

*Die Mengen URS(F$_i$), F$_i$ aus F, sollen sämtliche, diesen Mengen zugehörigen Ereignisse enthalten.*

Erläuterung zu Voraussetzung IIa

Auch bei nicht-computergestützten Verfahren leidet die Diagnose, wenn für einzelne Symptome nicht alle in Frage kommenden Ursachen berücksichtigt sind. Der vornehmliche Grund für die vorliegende Forderung ist jedoch, daß die Ereignisse $F_i$ mit Hilfe einer Bedingung stochastisch unabhängig werden können, wenn diese Bedingung sämtliche Elemente aus URS($F_i$) enthält.

*Voraussetzung IIb*

*Die Ereignisse in URS(F$_i$), F$_i$ aus F, sind selbstständige Ursachen der F$_i$.*

Erläuterung zu Voraussetzung IIb

Die Selbständigkeit von Ursachen ist für den nachfolgend eingeführten A.L-Satz exakt definiert. Anschaulich bedeutet es, daß für einen beliebigen Netzknoten L

- die Ereignisse in URS(L) stochastisch unabhängig sind, und daß
- ein beliebiger L-erzeugender Vorgang nicht durch andere Ursachen oder durch die Inhibitoren eines weiteren L-erzeugenden Vorgangs beeinflußt wird.

Im Fall medizinischer Anwendungen kann die Erfüllung der Voraussetzung IIb angenommen werden, wenn die einzelnen Ursachen als nicht "nahe verwandt" anzusehen sind.

*Voraussetzung IIc*

*Stochast. Unabhängigkeit gilt für die Elemente in URS($F_i$) $\cup$ INH($F_i$), $F_i$ aus F.*

Erläuterung zu Voraussetzung IIc

Für die $F_i$ aus F erreicht man Unabhängigkeit mittels eines Bedingungsverbundes, der alle Ursachen der $F_i$ enthält, wenn noch zudem alle Elemente in URS($F_i$) $\cup$ INH($F_i$) unabhängig sind.

*Voraussetzung III*

$$p(U\,F\,K\,I\,J|H) = p(UI|H)\,p(F\,K\,J|H)\ \text{für } H \text{ und } \bar{H}.$$

Erläuterung zu Voraussetzung III

Die Gleichung der Voraussetzung III ist die mathematische Formulierung der durch H bewirkten Separierung von (U I) und (F K J). Daraus ergibt sich für die Kausalstruktur, daß Elemente aus (U I) nur über H mit den Knoten des "darunter" liegenden Netzes verknüpft sein dürfen.

*Die Voraussetzungen IIb und IIc wurden separat formuliert,* da *sie im weiteren in diesen Formen verwendet werden. Es besteht jedoch die Entsprechung IIb $\Leftarrow$ IIc, und mit einer außerordentlich geringfügigen Zusatzbedingung auch IIb $\Leftrightarrow$ IIc.*

Ergänzende Voraussetzungen

Die Voraussetzungen IIa, IIb und IIc sind formuliert für URS($F_i$) und INH($F_i$), $F_i$ aus F. Völlig analoge Voraussetzungen können für URS(H) und INH(H) gefordert werden; sie seien mit IIa*, IIb* und IIc* bezeichnet.

5. Berechnung der Zutreffenswahrscheinlichkeiten

Mit den getroffenen Voraussetzungen ergibt sich für p(H|H') = p(H|U F K I J):

$$p(H\,|\,UFKIJ) = \frac{p(HUFKIJ)}{p(HUFKIJ) + p(\overline{H}UFKIJ)}$$

$$= \frac{1}{1 + \dfrac{p(UFKIJ\,|\,\overline{H})\,p(\overline{H})}{p(UFKIJ\,|\,H)\,p(H)}}$$

$$=_{(\text{Vorauss.III})} \frac{1}{1 + \dfrac{p(FKJ\overline{H})\,p(UI|\,\overline{H})}{p(FKJH)\,p(UI|\,H)}}$$

$$=_{(\text{Vorauss.IIc})} \frac{1}{1 + \dfrac{p(F\,|\,KJ\overline{H})\,p(\overline{H}\,|\,UI)}{p(F\,|\,KJH)\,p(H\,|\,UI)}}\ . \qquad (5.1)$$

Zur weiteren Bearbeitung wird p(F|K J H) linear interpoliert (die maßgebende Ziffer b) ist erfüllt, da alle entseparierenden $F_i$ vor dem Bedingungsstrich stehen). Dasselbe Ergebnis liefert der L-Satz, 5-fach angewendet auf die rechte Seite von: p(F$_1$...F$_5$|K J H) = p(F$_1$|F$_2$...F$_5$ K J H) p(F$_2$|F$_3$...F$_5$ K J H)· ...· p(F$_5$|K J H).

Mit Glg.3.3 (oder dem L-Satz) erhält man bei n '-Elementen K$_1$', ..., K$_n$':

$$p(F \mid K_1' \ldots K_n' \, J \, H) = \sum_{q_1,\ldots,q_n=0,1} p(F \mid K_1^{(q_1)}\ldots K_n^{(q_n)} JH) p(K_1^{(q_1)} \mid K_1') \cdot \ldots \cdot p(K_n^{(q_n)} \mid K_n').$$

Da der Bedingungsverbund sämtliche Ursachen der F$_i$ aus F enthält, und da die Elemente in INH(F$_1$) ∪ ...∪ INH(F$_5$) stochastisch unabhängig sind, folgt:

$$p(F \mid K_1^{(q_1)}\ldots K_n^{(q_n)} JH) = \prod_i p(F_i \mid K_1^{(q_1)}\ldots K_n^{(q_n)} JH), \, F_i \text{ aus } F . \qquad (5.1a)$$

Die Wahrscheinlichkeiten der Form p(Folge | Ursachen ∧ Inhibitoren) können unter Nutzung der Selbstständigkeitseigenschaft der Elemente in URS(F$_i$) und URS(H) weiter vereinfacht werden.

Sei hierzu:

L Beliebiges Ereignis.

A Beliebig gewählter, aber feststehender Verbund aus unnegierten Ereignissen von URS(L) mit A:= (A$_1$... A$_k$).

X Beliebiger Verbund mit negierten oder unnegierten Ereignissen aus URS(L) \ {Elemente in A}.

A→L Kausaler Vorgang "A erzeugt L" . Ist A ein Verbund mit A:= (A$_1$...A$_k$), so bedeutet A→L: "A$_1$ erzeugt L" ∪...∪ "A$_k$ erzeugt L".

D Beliebiger Verbund mit Ereignissen, die für A$_1$→L,..., A$_k$→L Inhibitoren sind, oder die Inhibition von A$_1$→L,..., A$_k$→L verstärken oder abschwächen.

*Definition (Selbständigkeit)*

*A heißt selbständige Ursache von L, falls A für alle X die nachfolgenden Voraussetzungen 1/A→L bis 4/A→L erfüllt:*

$$\textit{Voraussetzung 1/A} \rightarrow \textit{L: } p((A \rightarrow L)(X \rightarrow L) \mid A\,X) = p(A \rightarrow L \mid A\,X)\,p(X \rightarrow L \mid A\,X).$$

$$\textit{Voraussetzung 2/A} \rightarrow \textit{L: } \qquad p(A \rightarrow L \mid A\,X) = p(A \rightarrow L \mid A).$$

$$\textit{Voraussetzung 3/A} \rightarrow \textit{L: } \qquad p(X \rightarrow L \mid A\,X) = p(X \rightarrow L \mid \bar{A}_1\ldots\bar{A}_k X).$$

$$\textit{Voraussetzung 4/A} \rightarrow \textit{L: } \qquad p(X \mid A) = p(X \mid \bar{A}_1\ldots\bar{A}_k).$$

Damit ergibt sich:

**_Satz_**

*A→L-Satz und A→L-Korollar 1*

*Unter der Voraussetzung, daß A eine selbständige Ursache von L ist, gilt mit A:= (A$_1$...A$_k$):*

$$\textit{A} \rightarrow \textit{L-Satz:} \qquad p(A \rightarrow L \mid A) = \frac{p(L|A) - p(L|\bar{A}_1\ldots\bar{A}_k)}{p(\bar{L}|\bar{A}_1\ldots\bar{A}_k)}. \qquad (5.2)$$

$$A{\rightarrow}L\text{-Korollar 1:} \qquad p(A{\rightarrow}L \mid AD) = \frac{p(L|AD)\text{-}p(L|\bar{A}_1...\bar{A}_kD)}{p(\bar{L}|\bar{A}_1...\bar{A}_kD)}. \qquad\qquad (5.3)$$

Beweis: Literatur.

Mit dem A→L-Satz bzw. dem A→L-Korollar 1 lassen sich Wahrscheinlichkeiten der Form *p(Folge | Ursachen)* entscheidend vereinfachen.

### ***Satz***

*Ursachen-Faktorisierungssatz*

*Die Menge {H, $K_1$, ..., $K_n$} enthalte sämtliche Ursachen eines beliebigen Ereignisses $F_1$. Alle Ursachen sind selbständige Ursachen von $F_1$.*

*Exemplarisch seien*

$I_1, ... , I_r$ *die Inhibitoren von $K_1{\rightarrow}F_1$ und*
$J_1,..., J_s$ *die Inhibitoren von $K_2{\rightarrow}F_1$.*

*Dann gilt:*

$$
\begin{aligned}
p(\overline{F}_1 \mid H K_1 ... K_n I_1 ... I_r J_1 ... J_s) \;=\; & p(\overline{F}_1 \mid H\overline{K}_1...\overline{K}_n) \qquad\qquad (5.4)\\[4pt]
& \cdot\, p(\overline{F}_1 \mid \overline{H}K_1\overline{K}_2...\overline{K}_nI_1...I_r)\\[4pt]
& \cdot\, p(\overline{F}_1 \mid \overline{H}\cdot\overline{K}_1K_2\overline{K}_3...\overline{K}_nJ_1...J_s)\\[4pt]
& \cdot\, p(\overline{F}_1 \mid \overline{H}\cdot\overline{K}_1\overline{K}_2K_3\overline{K}_4...\overline{K}_n)\\[4pt]
& \;\;\vdots\\[4pt]
& \cdot\, p(\overline{F}_1 \mid \overline{H}\cdot\overline{K}_1...\overline{K}_{n-1}K_n).
\end{aligned}
$$

*Insbesondere gilt:*

$$(\bar{F}_1|HK_1 ... K_n)$$

$$= p(\bar{F}_1 \mid H\bar{K}_1...\bar{K}_n)p(\bar{F}_1|\bar{H}K_1\bar{K}_2...\bar{K}_n)\cdot.....\cdot p(\bar{F}_1|\bar{H}\cdot\bar{K}_1...\bar{K}_{n-1}K_n). \qquad\qquad (5.5)$$

Beweis:

$$p(F_1 \mid H\, K_1 \ldots K_n\, I_1 \ldots I_r\, J_1 \ldots J_s)$$

$$= p(\,(H \rightarrow F_1) \cup (K_1 \rightarrow F_1) \cup \ldots \cup (K_n \rightarrow F_1) \mid H\, K_1 \ldots K_n\, I_1 \ldots I_r\, J_1 \ldots J_s)$$

$$= 1 - p((\overline{H \rightarrow F_1}) \cap (\overline{K_1 \rightarrow F_1}) \cap \ldots \cap (\overline{K_n \rightarrow F_1}) \mid H K_1 \ldots K_n I_1 \ldots I_r J_1 \ldots J_s)$$

$$= 1 - [\,1 - p(H \rightarrow F_1 \mid H\, K_1 \ldots K_n\, I_1 \ldots I_r\, J_1 \ldots J_s)]$$

$$\cdot [\,1 - p(K_1 \rightarrow F_1 \mid H\, K_1 \ldots K_n\, I_1 \ldots I_r\, J_1 \ldots J_s)]$$

$$\vdots$$

$$\cdot [\,1 - p(K_n \rightarrow F_1 \mid H\, K_1 \ldots K_n\, I_1 \ldots I_r\, J_1 \ldots J_s)]$$

$$= 1 - [\,1 - p(H \rightarrow F_1 \mid H\, \overline{K}_1 \ldots \overline{K}_n)] \qquad \text{(wg. Selbständigkeit)}$$

$$\cdot [\,1 - p(K_1 \rightarrow F_1 \mid \overline{H} K_1 \overline{K}_2 \ldots \overline{K}_n\, I_1 \ldots I_r)]$$

$$\cdot [\,1 - p(K_2 \rightarrow F_1 \mid \overline{H} \cdot \overline{K}_1 K_2 \overline{K}_3 \ldots \overline{K}_n\, J_1 \ldots J_s)]$$

$$\cdot [\,1 - p(K_3 \rightarrow F_1 \mid \overline{H} \cdot \overline{K}_1 \overline{K}_2 K_3 \overline{K}_4 \ldots \overline{K}_n)]$$

$$\vdots$$

$$\cdot [\,1 - p(K_n \rightarrow F_1 \mid \overline{H} \cdot \overline{K}_1 \ldots \overline{K}_{n-1} K_n)]$$

$$= \quad (\text{A.L.-Satz bzw. A.L.-Korollar 1})$$

$$= 1 - \left[\,1 - \frac{p(F_1 \mid H \overline{K}_1 \ldots \overline{K}_n) - p(F_1 \mid \overline{H} \cdot \overline{K}_1 \ldots \overline{K}_n)}{1 - p(F_1 \mid \overline{H} \cdot \overline{K}_1 \ldots \overline{K}_n)}\,\right]$$

$$\cdot \left[\,1 - \frac{p(F_1 \mid \overline{H} K_1 \overline{K}_2 \ldots \overline{K}_n I_1 \ldots I_r) - p(F_1 \mid \overline{H} \cdot \overline{K}_1 \ldots \overline{K}_n I_1 \ldots I_r)}{1 - p(F_1 \mid \overline{H} \cdot \overline{K}_1 \ldots \overline{K}_n I_1 \ldots I_r)}\,\right]$$

$$\cdot \left[\,1 - \frac{p(F_1 \mid \overline{H} \cdot \overline{K}_1 K_2 \overline{K}_3 \ldots \overline{K}_n J_1 \ldots J_s) - p(F_1 \mid \overline{H} \cdot \overline{K}_1 \ldots \overline{K}_n J_1 \ldots J_s)}{1 - p(F_1 \mid \overline{H} \cdot \overline{K}_1 \ldots \overline{K}_n J_1 \ldots J_s)}\,\right]$$

$$\cdot \left[\,1 - \frac{p(F_1 \mid \overline{H} \cdot \overline{K}_1 \overline{K}_2 K_3 \overline{K}_4 \ldots \overline{K}_n) - p(F_1 \mid \overline{H} \cdot \overline{K}_1 \ldots \overline{K}_n)}{1 - p(F_1 \mid \overline{H} \cdot \overline{K}_1 \ldots \overline{K}_n)}\,\right]$$

$$\vdots$$

$$\cdot \left[\,1 - \frac{p(F_1 \mid \overline{H} \cdot \overline{K}_1 \ldots \overline{K}_{n-1} K_n) - p(F_1 \mid \overline{H} \cdot \overline{K}_1 \ldots \overline{K}_n)}{1 - p(F_1 \mid \overline{H} \cdot \overline{K}_1 \ldots \overline{K}_n)}\,\right]$$

$$= \quad (\text{nächste Seite})$$

$$= 1 - \frac{p(\overline{F}_1 \mid H \overline{K}_1 ... \overline{K}_n)}{p(\overline{F}_1 \mid \overline{H} \cdot \overline{K}_1 ... \overline{K}_n)}$$

$$\cdot \frac{p(\overline{F}_1 \mid \overline{H} K_1 \overline{K}_2 ... \overline{K}_n I_1 ... I_r)}{p(\overline{F}_1 \mid \overline{H} \cdot \overline{K}_1 ... \overline{K}_n)}$$

$$\cdot \frac{p(\overline{F}_1 \mid \overline{H} \cdot \overline{K}_1 K_2 \overline{K}_3 ... \overline{K}_n J_1 ... J_s)}{p(\overline{F}_1 \mid \overline{H} \cdot \overline{K}_1 ... \overline{K}_n)}$$

$$\cdot \frac{p(\overline{F}_1 \mid \overline{H} \cdot \overline{K}_1 \overline{K}_2 K_3 \overline{K}_4 ... \overline{K}_n)}{p(\overline{F}_1 \mid \overline{H} \cdot \overline{K}_1 ... \overline{K}_n)}$$

$$\vdots$$

$$\cdot \frac{p(\overline{F}_1 \mid \overline{H} \cdot \overline{K}_1 ... \overline{K}_{n-1} K_n)}{p(\overline{F}_1 \mid \overline{H} \cdot \overline{K}_1 ... \overline{K}_n)} \ .$$

Die letzte Gleichungszeile ergibt sich unter der Beachtung, daß Inhibitoren ohne Bedeutung für ein Folge-Ereignis sind , wenn die zugehörige Ursache negiert vorliegt.

Mit $p(\overline{F}_1 \mid \overline{H} \cdot \overline{K}_1 ... \overline{K}_n) = 1$ folgt die Aussage. Q.e.d.

Der Ursachen-Faktorisierungssatz erweist sich als außerordentlich praktisch. Eine Faktorisierung der Wahrscheinlichkeit $p(\overline{F}_1 \mid H K_1 ... K_n)$ kann unmittelbar angeschrieben werden.

Bemerkenswert ist dann aber auch das durch diese Aktion erreichte Produkt $p(\overline{F}_1|H\overline{K}_1...\overline{K}_n)_p(\overline{F}_1|\overline{H}K_1\overline{K}_2...\overline{K}_n)\cdot .....\cdot p(\overline{F}_1|\overline{H}\cdot\overline{K}_1...\overline{K}_{n-1}K_n)$.

Jeder Bedingungsverbund der enthaltenen Wahrscheinlichkeiten besitzt genau eine mit p = 1 vorliegende Ursache. Dadurch werden repräsentative Stichproben zur Ermittlung der jeweils zugehörigen relativen Häufigkeiten sehr vereinfacht. Im Fall der ersten Wahrscheinlichkeit bedeutet es, daß man eine Population mit der Eigenschaft H heranzieht und dabei sicherstellt, daß alle weiteren Ursachen $K_1$ bis Kn nicht vorliegen. Sodann sind auf dieser Population die Fälle mit dem Folge-Ereignis $F_1$ abzuzählen, und abschließend ist nur noch $1- h(F_1|H \overline{K}_1...\overline{K}_n)$ zu bilden.

Bisher wurde eine Faktorisierung bzgl. der Inhibitoren außer Acht gelassen, die man jedoch auch durchführen kann. Dazu sei $F_1$ ein beliebiger Netzknoten, $K_1$ sei ein beliebig gewähltes Element aus URS($F_1$), und die Ereignisse $I_1, ..... I_r$ seien die Inhibitoren von $K_1{\rightarrow}F_1$.

*Voraussetzung für* Inhibitoren

*Unter der Bedingung des Vorliegens von $K_1$, sind die Inhibitoren $I_1, ..., I_r$ selbständige Ursachen des Ereignisses $\overline{(K1 \rightarrow F_1)}$.*

Erläuterung zur Voraussetzung für Inhibitoren

Für die $I_1, ..., I_r$, als selbständige Ursachen des Ereignisses $\overline{(K_1 \rightarrow F_1)}$ unter der Bedingung $K_1$, gilt das A→L-Korollar 1 unverändert. Für die dort verwendeten Bezeichnungen gelten folgende Entsprechungen:

| | A→L-Korollar 1 | Faktorisierungssatz für Inhibitoren |
|---|---|---|
| Folge-Ereignis | L | $\overline{(K1 \rightarrow F_1)}$ |
| Ursachenmenge | URS(L) | $\{ I_1, ..., I_r \}$. |

Die $I_1, ..., I_r$ sind stochastisch unabhängig unter der Bedingung $K_1$.

Nach dem A→L-Korollar 2 wird die stochastische Unabhängigkeit zweier Ereignisse $I_1$ und $I_2$ durch ein bedingendes, negiertes, gemeinsames Folge-Ereignis nicht aufgehoben; nicht aufgehoben in diesem Fall also durch das doppelt negierte Ereignis $\overline{(K_1 \rightarrow \overline{F}_1)} = (K_1{\rightarrow}F_1)$.

Die unter der Bedingung $K_1$ unabhängigen Ereignisse $I_1$, ..., $I_r$ sind also auch unter der Bedingung ($K_1$ ($K_1 \rightarrow F_1$)) unabhängig.

### *Satz*

### *Inhibitoren-Faktorisierungssatz*

*Sei $F_1$ ein beliebiger Netzknoten, $K_1$ sei eine selbständige Ursache von $F_1$, und $I_1,...,I_r$ seien die Inhibitoren von $K_1 \rightarrow F_1$.*

*Unter der Voraussetzung, daß die Inhibitoren $I_1,..., I_r$ selbständige Ursachen des Ereignisses $(\overline{K1 \rightarrow F_1})$ unter der Bedingung $K_1$ sind, gilt:*

$$p(K_1 \rightarrow F_1 | K_1\, I_1\, ...\, I_r) =$$

$$p(K_1 \rightarrow F_1 | K_1) \cdot \frac{p(K_1 \rightarrow F_1 | K_1 I_1)}{p(K_1 \rightarrow F_1 | K_1)} \cdot ..... \cdot \frac{p(K_1 \rightarrow F_1 | K_1 I_r)}{p(K_1 \rightarrow F_1 | K_1)}. \tag{5.6}$$

*Bezeichnen zusätzlich*

$$i_1 := \frac{p(F_1 | K_1 I_1 \overline{H} \cdot \overline{K}_2 ... \overline{K}_n)}{p(F_1 | K_1 \overline{H} \cdot \overline{K}_2 ... \overline{K}_n)},\ ...\ ,\ i_r := \frac{p(F_1 | K_1 I_r \overline{H} \cdot \overline{K}_2 ... \overline{K}_n)}{p(F_1 | K_1 \overline{H} \cdot \overline{K}_2 ... \overline{K}_n)},$$

dann gilt:

$$(p(K_1 \rightarrow F_1 | K_1 I_1\, ... I_r) = p(K_1 \rightarrow F_1 | K_1) \cdot i_1 \cdot\ ... \cdot i_r. \tag{5.7}$$

Beweis:

Das $A \rightarrow L$-Korollar 2 sagt aus, daß zwei stochastisch unabhängige Ereignisse A und B, die selbständige Ursachen eines Ereignisses L sind, unter der Bedingung $\overline{L}$ unabhängig bleiben. Damit sind die $I_1,..., I_r$ unter der Bedingung $\overline{(K_1 \rightarrow F_1)}$, also auch unter der Bedingung ($K_1$ ($K_1 \rightarrow F_1$)) stochastisch unabhängig.

Es folgt:

$$p(K_1 \rightarrow F_1 \mid K_1\, I_1\, ...\, I_r)$$

$$= \frac{p(I_1 ... I_r \mid K_1 (K_1 \rightarrow F_1)) \cdot p(K_1 (K_1 \rightarrow F_1))}{p(K_1) p(I_1) \cdot ... \cdot p(I_r)}$$

$$= \frac{p(I_1 \mid K_1 (K_1 \rightarrow F_1))}{p(I_1)} \cdot ..... \cdot \frac{p(I_r \mid K_1 (K_1 \rightarrow F_1))}{p(I_r)} \cdot p(K_1 \rightarrow F_1 \mid K_1)$$

$$= \frac{p(K_1 \rightarrow F_1 \mid K_1 I_1)}{p(K_1 \rightarrow F_1 \mid K_1)} \cdot ..... \cdot \frac{p(K_1 \rightarrow F_1 \mid K_1 I_r)}{p(K_1 \rightarrow F_1 \mid K_1)} \cdot p(K_1 \rightarrow F_1 \mid K_1).$$

Damit ist die Glg.5.6 bewiesen.

Die Gültigkeit der Glg.5.7 wird exemplarisch für den ersten Quotienten gezeigt:

$$\frac{p(K_1 \to F_1 \mid K_1 I_1)}{p(K_1 \to F_1 \mid K_1)}$$

$$= \frac{p(K_1 \to F_1 \mid K_1 I_1 \overline{H} \cdot \overline{K}_2...\overline{K}_n)}{p(K_1 \to F_1 \mid K_1 \overline{H} \cdot \overline{K}_2...\overline{K}_n)}$$

$$= \quad (\text{wegen A} \to \text{L-Korollar 1})$$

$$= \frac{p(F_1 \mid K_1 I_1 \overline{H} \cdot \overline{K}_2...\overline{K}_n) - p(F_1 \mid \overline{K}_1 I_1 \overline{H} \cdot \overline{K}_2...\overline{K}_n)}{p(\overline{F}_1 \mid \overline{K}_1 I_1 \overline{H} \cdot \overline{K}_2...\overline{K}_n)}$$

$$\cdot \frac{p(\overline{F}_1 \mid \overline{K}_1 \overline{H} \cdot \overline{K}_2...\overline{K}_n)}{p(F_1 \mid K_1 \overline{H} \cdot \overline{K}_2...\overline{K}_n) - p(F_1 \mid \overline{K}_1 \overline{H} \cdot \overline{K}_2...\overline{K}_n)}$$

$$= \quad (\text{ wegen } p(F_1 \mid \overline{K}_1 I_1) = p(F_1 \mid \overline{K}_1))$$

$$= \frac{p(F_1 \mid K_1 I_1 \overline{H} \cdot \overline{K}_2...\overline{K}_n) - p(F_1 \mid \overline{H} \cdot \overline{K}_1...\overline{K}_n)}{p(F_1 \mid K_1 \overline{H} \cdot \overline{K}_2...\overline{K}_n) - p(F_1 \mid \overline{H} \cdot \overline{K}_1...\overline{K}_n)}$$

$$= \quad (\text{ wg. } p(F_1 \mid \overline{H} \cdot \overline{K}_1...\overline{K}_n) = 0)$$

$$= \frac{p(F_1 \mid K_1 I_1 \overline{H} \cdot \overline{K}_2...\overline{K}_n)}{p(F_1 \mid K_1 \overline{H} \cdot \overline{K}_2...\overline{K}_n)}, \text{q.e.d.}$$

### *Satz*

*Kombinierter Faktorisierungssatz*

*Die Menge {H, $K_1$, ..., $K_n$} enthalte sämtliche Ursachen eines beliebigen Ereignisses $F_1$. Alle Ursachen sind selbständige Ursachen von $F_1$. Exemplarisch seien $I_1, ... , I_r$ die Inhibitoren von $K_1 \to F_1$ und $J_1, ... , J_s$ die Inhibitoren von $K_2 \to F_1$.*
*Zusätzlich zu den Bezeichnungen des Inhibitoren-Faktorisierungssatzes sei*

$$j_1 := \frac{p(F_1 \mid \overline{H} \cdot \overline{K}_1 K_2 \overline{K}_3...\overline{K}_n J_1)}{p(F_1 \mid \overline{H} \cdot \overline{K}_1 K_2 \overline{K}_3...\overline{K}_n)}, \, .... \, , j_s := \frac{p(F_1 \mid \overline{H} \cdot \overline{K}_1 K_2 \overline{K}_3...\overline{K}_n J_s)}{p(F_1 \mid \overline{H} \cdot \overline{K}_1 K_2 \overline{K}_3...\overline{K}_n)},$$

dann gilt:

$$p(\overline{F}_1 \mid H\,K_1 \dots K_n\,I_1 \dots I_r\,J_1 \dots J_s) \;=\; p(\overline{F}_1 \mid H\overline{K}_1 \dots \overline{K}_n)$$

$$\cdot [\,1 - p(F_1 \mid \overline{H}K_1\overline{K}_2 \dots \overline{K}_n)\cdot i_1 \dots i_r\,]$$

$$\cdot [\,1 - p(F_1 \mid \overline{H}\cdot\overline{K}_1 K_2 \overline{K}_3 \dots \overline{K}_n)\cdot j_1 \dots j_s\,]$$

$$\cdot p(F_1 \mid \overline{H}\cdot\overline{K}_1\cdot\overline{K}_2 K_3 \overline{K}_4 \dots \overline{K}_n)$$

$$\vdots$$

$$\cdot p(\overline{F}_1 \mid \overline{H}\cdot\overline{K}_1 \dots \overline{K}_{n-1} K_n)\,.$$

*Beweis:*

$$p(F_1 \mid H\,K_1 \ldots K_n\,I_1 \ldots I_r\,J_1 \ldots J_s)$$

$$= p((H \to F_1) \cup (K_1 \to F_1) \cup \ldots \cup (K_n \to F_1) \mid H\,K_1 \ldots K_n\,I_1 \ldots I_r\,J_1 \ldots J_s)$$

$$= 1 - p((\overline{H \to F_1}) \cap (\overline{K_1 \to F_1}) \cap \ldots \cap (\overline{K_n \to F_1}) \mid H K_1 \ldots K_n I_1 \ldots I_r, J_1 \ldots J_s)$$

$$= 1 - [\,1 - p(H \to F_1 \mid H\,K_1 \ldots K_n\,I_1 \ldots I_r\,J_1 \ldots J_s)]$$
$$\cdot [\,1 - p(K_1 \to F_1 \mid H\,K_1 \ldots K_n\,I_1 \ldots I_r\,J_1 \ldots J_s)]$$
$$\vdots$$
$$\cdot [\,1 - p(K_n \to F_1 \mid H\,K_1 \ldots K_n\,I_1 \ldots I_r\,J_1 \ldots J_s)]$$

$$= 1 - [\,1 - p(H \to F_1 \mid H\,\overline{K}_1 \ldots \overline{K}_n)]$$
$$\cdot [\,1 - p(K_1 \to F_1 \mid \overline{H} K_1 \overline{K}_2 \ldots \overline{K}_n\,I_1 \ldots I_r)]$$
$$\cdot [\,1 - p(K_2 \to F_1 \mid \overline{H} \cdot \overline{K}_1 K_2 \overline{K}_3 \ldots \overline{K}_n\,J_1 \ldots J_s)]$$
$$\cdot [\,1 - p(K_3 \to F_1 \mid \overline{H} \cdot \overline{K}_1 \overline{K}_2 K_3 \overline{K}_4 \ldots \overline{K}_n)]$$
$$\vdots$$
$$\cdot [\,1 - p(K_n \to F_1 \mid \overline{H} \cdot \overline{K}_1 \ldots \overline{K}_{n-1} K_n)]$$

$$= 1 - [\,1 - p(H \to F_1 \mid H\,\overline{K}_1 \ldots \overline{K}_n)]$$
$$\cdot [\,1 - p(K_1 \to F_1 \mid \overline{H} K_1 \overline{K}_2 \ldots \overline{K}_n) \cdot i_1 \ldots \cdot i_r)]$$
$$\cdot [\,1 - p(K_2 \to F_1 \mid \overline{H} \cdot \overline{K}_1 K_2 \overline{K}_3 \ldots \overline{K}_n) \cdot j_1 \ldots \cdot j_s)]$$
$$\cdot [\,1 - p(K_3 \to F_1 \mid \overline{H} \cdot \overline{K}_1 \overline{K}_2 K_3 \overline{K}_4 \ldots \overline{K}_n)]$$
$$\vdots$$
$$\cdot [\,1 - p(K_n \to F_1 \mid \overline{H} \cdot \overline{K}_1 \ldots \overline{K}_{n-1} K_n)]$$

$$= \; 1 - \; p(\overline{F}_1 \mid H\,\overline{K}_1...\overline{K}_n)$$

$$\cdot [\,1 - \; p(F_1 \mid \overline{H}K_1\overline{K}_2...\overline{K}_n) \cdot i_1...i_r\,]$$

$$\cdot [\,1 - \; p(F_1 \mid \overline{H} \cdot \overline{K}_1 K_2 \overline{K}_3...\overline{K}_n) \cdot j_1...j_s\,]$$

$$\cdot \, p(\overline{F}_1 \mid \overline{H} \cdot \overline{K}_1 \overline{K}_2 K_3 \overline{K}_4...\overline{K}_n)$$

$$\vdots$$

$$\cdot \, p(\overline{F}_1 \mid \overline{H} \cdot \overline{K}_1...\overline{K}_{n-1} K_n)\,, \, \text{q.e.d.}$$

Auf der rechten Seite der Gleichung in Anspruch 1 ist außer p(F | K J H) auch p(H | U I) zu bearbeiten.

p(H | UI) wird ebenfalls linear interpoliert, da die Ereignisse in (U I) die Ziffer b) des Linearen Interpolationssatzes erfüllen. Die entstehenden Wahrscheinlichkeiten besitzen die Form p(H|$U_1$ ... $U_3$ $I_1$ ...$I_2$).

Somit liefern sowohl p(F|K J H) als auch p(H | U I) bedingte Wahrscheinlichkeiten mit der Grundstruktur *p (Folge | Ursachen $\wedge$ Inhibitoren)*, die mit Hilfe der angegebenen Sätze in Wahrscheinlichkeiten kleineren Umfangs zerlegt werden können.

<u>6. Berechnung eines Kausalnetzes am Beispiel der Abb.2. 1</u>

Eine Kausalstruktur wie in Abb.2.1 wird über die '-gekennzeichneten Ereignisse der H/K-Ebene fortgesetzt. Dieses kann so erfolgen, daß für jedes dieser Ereignisse modulartig ein L-Netz erstellt wird, wie es mit dem Beispiel der Abb.2.1 für H demonstriert ist. Die einzelnen Module sind übersichtlicher und bilden zusammengesetzt die Gesamt-Kausalstruktur.

Für das Berechnungsbeispiel seien $R_1$ und $R_3$ die <u>Re</u>präsentanten solcher modulartigen Fortsetzungen, z. B. $R_1$ aus dem L-Netz-Modul für $K_1$ und $R_3$ aus dem L-Netz-Modul für $K_3$, wobei exemplarisch $R_1 \in$ URS($K_1$) und $R_3 \in$ URS($K_3$) gewählt werden kann.

<u>Schritt 1</u>

Für die erste Verdachtshypothese H wird ein L-Netz erstellt, das die Ereignismengen URS(H), FOL(H), DIFF (H), INH(H) und

$$\bigcup_{Z \in FOL(H)} INH(Z)$$

enthält, wobei Vollzähligkeit für die Menge DIFF(H) in jedem Fall (Voraussetzung IIa), und für die Menge URS(H) im Bedarfsfall (Voraussetzung IIa*) zu fordern ist.

Insgesamt sind für die Strukturierung des L-Netzes die Voraussetzungen I, IIa, IIc und III, sowie IIa* und IIc* maßgebend.

Es interessieren die Zutreffenswahrscheinlichkeiten der '-gekennzeichneten Ereignisse in der H/K-Ebene. Für jedes dieser Ereignisse wird nach denselben Grundsätzen wie für H ein L-Netz-Modul erstellt.

<u>Vorteil gegenüber anderen Systemen</u>

Nicht zufriedenstellend gelöst war bisher das Problem, daß sich die Zutreffenswahrscheinlichkeiten der "-gekennzeichneten Ereignisse eines Kausalnetzes wechselseitig beeinflussen. Durch die gleichzeitige Erfassung aller unbekannten Zutreffenswahrscheinlichkeiten in einem Gleichungssystem (wie es nachfolgend vorgestellt wird) ist diese Schwierigkeit behoben.

<u>Schritt 2</u>

Für jedes '-gekennzeichnete Ereignis des Kausalnetzes wird unter Verwendung der Glg.2.2 eine sogenannte Wertungsgleichung erstellt. Man erhält das folgende Gleichungssystem der Glgn. 6.1 bis 6.5:

$$p(H \mid H') \quad = \quad p(H \mid W(H))$$
$$= \quad p(H \mid U\ F\ K\ I\ J)$$
$$= \quad (\ \text{ident. Glg.2.3}). \tag{6.1}$$

$$p(K_1 \mid K_1') = p(K_1 \mid W(K_1))$$
$$= p(K_1 \mid F_1\ F_2\ H'\ J_1\ R_1). \tag{6.2}$$

$$p(K_3 \mid K_3') = p(K_3 \mid W(K_3))$$
$$= p(K_3 \mid F_5\ H'\ \overline{J}_2\ R_3). \tag{6.3}$$

$$p(U_2 \mid U_2') = p(U_2 \mid W(U_2))$$
$$= p(U_2 \mid H'\ U_1\ U_3'\ I_1\ I_2). \tag{6.4}$$

$$p(U_3 \mid U_3') = p(U_3 \mid W(U_3))$$
$$= p(U_3 \mid H'\ U_1\ U_2'\ I_1\ I_2). \tag{6.5}$$

Man wird in praxi den Aufwand für p($U_2 \mid U_2'$) und p($U_3 \mid U_3'$), oder gar für weiter "darüber" liegende Ereignisse, möglichst gering halten.

Es sollte jedoch (U I) $\neq$ Ø sein, um in Glg.5.1 die schwer zu ermittelnde a-priori-Wahrscheinlichkeit p(H) zu vermeiden. Auch aus diesem Grund interpoliert man die Glgn.6.4 und 6.5 mit maximalem $V_{E'_i}$, so daß $p(E_i \mid V_{E'_i}) \neq p(E_i)$ gilt.

Schritt 3

Man erhält bei Anwendung der Glg.3.1 bzw. der Glg.5.1 das Gleichungssystem der Glgn.6.1a bis 6.5a wie folgt:

$$p(H|H') = {}_{(\text{Glg.5.1})} \frac{1}{1 + \dfrac{p(F_1...F_5|K'_1 K_2 K'_3 J_1 \bar{J}_2 \overline{H})p(\overline{H}|UI)}{p(F_1...F_5|K'_1 K_2 K'_3 J_1 \bar{J}_2 H)p(H|UI)}}. \tag{6.1a}$$

In Glg.6.1a ist:

$$p(F_1...F_5 \mid K_1^{'}K_2K_3^{'}J_1\overline{J}_2H)$$

$$= \sum_{q_1,q_3=0,1} p(F_1...F_5 \mid K_1^{(q_1)}K_2K_3^{(q_3)}J_1\overline{J}_2H)p(K_1^{(q_1)} \mid K_1^{'})p(K_3^{(q_3)} \mid K_3^{'}).$$

In dieser Gleichung wiederum ist

$$p(F_1...F_5 \mid K_1^{(q_1)}K_2K_3^{(q_3)}J_1\overline{J}_2H) = \prod_{i=1}^{5} p(F_i \mid K_1^{(q_1)}K_2K_3^{(q_3)}J_1\overline{J}_2H),$$

wobei gilt:

$$p(F_1|K_1^{(q_1)}K_2K_3^{(q_3)}J_1\bar{J}_2H) = p(F_1|K_1^{(q_1)}H),$$

$$p(F_2|K_1^{(q_1)}K_2K_3^{(q_3)}J_1\bar{J}_2H) = p(F_2|K_1^{(q_1)}J_1H),$$

$$p(F_3|K_1^{(q_1)}K_2K_3^{(q_3)}J_1\bar{J}_2H) = p(F_3|K_2H),$$

$$p(\bar{F}_4|K_1^{(q_1)}K_2K_3^{(q_3)}J_1\bar{J}_2H) = p(\bar{F}_4|K_2H),$$

$$p(F_5|K_1^{(q_1)}K_2K_3^{(q_3)}J_1\bar{J}_2H) = p(F_5|K_3^{(q_3)}\bar{J}_2H).$$

Zudem ist in Glg.6.1a:

$$p(H \mid U \, I) = p(H \mid U_1 U_2{'} U_3{'} I_1 I_2)$$

$$= (\text{ident. Glg.3.4}).$$

$$p(K_1|K_1') =_{(\text{Glg.5.1})} \frac{1}{1+\dfrac{p(F_1F_2|H'J_1\bar{K}_1)p(\bar{K}_1|R_1)}{p(F_1F_2|H'J_1K_1)p(K_1|R_1)}}. \qquad (6.2a)$$

(Analog Glg.6.1a; ebenso die übrigen Ereignisse der H/K-Ebene.)

$$p(K_3|K_3') =_{(\text{Glg.5.1})} \frac{1}{1+\dfrac{p(F_5|H'\bar{J}_2\bar{K}_3)p(\bar{K}_3|R_3)}{p(F_5|H'\bar{J}_2K_3)p(K_3|R_3)}}. \qquad (6.3a)$$

$$p(U_2|U_2') =_{(\text{Glg.3.1})} (\text{ident.Glg.3.2}). \qquad (6.4a)$$

$$p(U_3|U_3') =_{(\text{Glg.3.1})} (\text{wie Glg.6.4a; } U_2, U_3 \text{ vertauscht}). \qquad (6.5a)$$

Schritt 4

Die nicht-bedingten Wahrscheinlichkeiten der Glgn.6.4a und 6.5a (vergl. dazu die ausgeschriebene Form der Glg.3.2) werden mittels einfacher Umformungen, z.B.

$$p(U_2 \, U_1 \, I_1 \, I_2 \, H \, U_3) = {}_{(IIc^*)} \, p(H \mid U_1 \, U_2 \, U_3 \, I_1 \, I_2) \, p(U_1) \, p(U_2) \, p(U_3) \, p(I_1) \, p(I_2),$$

in die für den nachstehenden Schritt 5 erforderliche Form gebracht.

Schritt 5

Bei Abwesenheit unbekannter Ursachen ergibt sich für die in Glg.6.1a benannten Faktoren ( z.B. für $q_1 = q_3 = 1$) nach dem Ursachen-Faktorisierungssatz:

$$p(F_1 \mid H \, K_1) = 1 - p(\bar{F}_1 \mid H \, \bar{K}_1) p(\bar{F}_1 \mid K_1 \bar{H}).$$

$$p(F_2 \mid K_1 \, J_1 \, H) = 1 - p(\bar{F}_2 \mid HJ_1 \bar{K}_1) p(\bar{F}_2 \mid K_1 \bar{H}).$$

$$p(F_3 \mid K_2 H). = 1 - p(\bar{F}_3 \mid H \, \bar{K}_2) p(\bar{F}_3 \mid K_2 \bar{H}).$$

$$\begin{aligned}
p(\overline{F}_4 \mid K_2 H) \;&=\; 1 - p(F_4 \mid K_2 \, H) \\
&=\; 1 - [\, 1 - p(\overline{F}_4 \mid H\overline{K}_2) p(\overline{F}_4 \mid K_2 \overline{H}) \,] \\
&=\; p(\overline{F}_4 \mid H\overline{K}_2) p(\overline{F}_4 \mid K_2 \overline{H}).
\end{aligned}$$

$$p(F_5 \mid K_3 J_2 H) = 1 - p(\bar{F}_5 \mid H \, \bar{K}_3) p(\bar{F}_5 \mid K_3 J_2 \bar{H}).$$

Somit ergibt sich ( bei $q_1 = q_3 = 1$):

$$\begin{aligned}
p(F_1 ... F_5 \mid K_2 J_1 \overline{J}_2 HK_1 K_3) \;&=\; \prod_{i=1}^{5} p(F_i \mid K_2 J_1 \overline{J}_2 HK_1 K_3) \\
&=\; [1 - p(\overline{F}_1 \mid H\overline{K}_1) p(\overline{F}_1 \mid K_1 \overline{H})] \\
&\quad \cdot [1 - p(\overline{F}_2 \mid HJ_1 \overline{K}_1) p(\overline{F}_2 \mid K_1 \overline{H})] \\
&\quad \cdot [1 - p(\overline{F}_3 \mid H\overline{K}_2) p(\overline{F}_3 \mid K_2 \overline{H})] \\
&\quad \cdot p(\overline{F}_4 \mid H\overline{K}_2) p(\overline{F}_4 \mid K_2 \overline{H}) \\
&\quad \cdot [1 - p(\overline{F}_5 \mid H\overline{K}_3) p(\overline{F}_5 \mid K_3 \overline{J}_2 \overline{H})].
\end{aligned}$$

Ganz entsprechend geht man bei den übrigen Gleichungen vor; die einzelnen Faktorisierungen sind mit Hilfe der zugehörigen Faktorisierungssätze sofort verfügbar.

Schritt 6

Mit den Glgn.6.1 bis 6.5 und den nachfolgenden Berechnungsschritten steht somit ein nicht-lineares Gleichungssystem in den Unbekannten p(H | H'), p(K$_1$|K$_1$'), p(K$_3$|K$_3$'), p(U$_2$|U$_2$') und p(U$_3$|U$_3$') zur Verfügung, das iterativ mit Hilfe des nicht-linearen Einzel- oder Gesamtschrittverfahrens gelöst wird. Hierbei kann im Startvektor für eine beliebige Unbekannte p(Y|Y') derjenige Zahlenwert verwendet werden, den man (z.B. im Fall von Anwendungen in der Medizin) durch eine nicht-computergestützte Diagnostik erhalten würde. Der Startvektor bildet so die erste Näherung für das gestellte Problem.

*7. Näherungen*

Bei Anwendung der Faktorisierungssätze wird stets vorausgesetzt, daß keine verdeckten und damit unberücksichtigten Ursachen existieren. Ist jedoch (in der Terminologie der voranstehenden Sätze) $p(F_1|\bar{H}\cdot\bar{K}_1...\bar{K}_n) > 0$, und wendet man dennoch die Faktorisierungssätze an, so ist das erreichte Ergebnis als eine Näherung anzusehen. Die Abweichung der solcherart bestimmten Werte vom jeweiligen wahren Wert, d.h. vom Wert bei Gültigkeit von $p(F_1|\bar{H}\cdot\bar{K}_1...\bar{K}_n) = 0$, kann abgeschätzt werden.

***Satz***

*Ursachen-Faktorisierungssatz bei nicht vollständiger Ursachenmenge*

*Die Menge {H, K$_1$, ..., K$_n$} enthalte sämtliche bekannten Ursachen eines beliebigen Ereignisses F$_1$. Es existieren unbekannte Ursachen von F$_1$, die unter der Be zeichnung K$_y$ zusammengefaßt werden. Alle Ursachen sind selbständige Ursachen von F$_1$.*

*Exemplarisch seien*

*I$_1$, ... , I$_r$ die Inhibitoren von K$_1\to$F$_1$ und*
*J$_1$, ... , J$_s$ die Inhibitoren von K$_2\to$F$_1$.*

*Dann gilt:*

$$p(\overline{F}_1 \mid HK_1...K_nI_1...I_r J_1...J_s) \;=\; p(\overline{F}_1 \mid H\overline{K}_1...\overline{K}_n) \qquad (7.1)$$

$$\cdot\, p(\overline{F}_1 \mid \overline{H}K_1\overline{K}_2...\overline{K}_nI_1...I_r)$$

$$\cdot\, p(\overline{F}_1 \mid \overline{H}\cdot\overline{K}_1K_2\overline{K}_3...\overline{K}_nJ_1...J_s)$$

$$\cdot\, p(\overline{F}_1 \mid \overline{H}\cdot\overline{K}_1\overline{K}_2K_3\overline{K}_4...\overline{K}_n)$$

$$\vdots$$

$$\cdot\, p(\overline{F}_1 \mid \overline{H}\cdot\overline{K}_1...\overline{K}_{n-1}K_n) \cdot f,$$

*mit*

$$f \approx \frac{1}{(p(\bar{F}_1|\bar{H}\cdot\bar{K}_1...\bar{K}_n))^{n+1}}, \; (n+1) := \text{Anzahl der Ursachen.}$$

*Insbesondere gilt:*

$$(\bar{F}_1|H K_1... K_n)$$

$$= p(\bar{F}_1|H \bar{K}_1...\bar{K}_n)p(\bar{F}_1|\bar{H} K_1\bar{K}_2...\bar{K}_n)\cdot.....\cdot p(\bar{F}_1|\bar{H}\cdot\bar{K}_1...\bar{K}_{n-1}K_n)\cdot f. \qquad (7.2)$$

Beweis:

$$p(F_1 \mid H\,K_1 \ldots K_n\,I_1 \ldots I_r\,J_1 \ldots J_s)$$

$$= p(\,(H{\to}F_1) \cup (K_1{\to}F_1) \cup \ldots \cup (K_n{\to}F_1) \cup (K_y{\to}F_1) \mid H\,K_1 \ldots K_n\,I_1 \ldots I_r\,J_1 \ldots J_s)$$

$$= 1 - p((\overline{H \to F_1}) \cap \ldots \cap (\overline{K_n \to F_1}) \cap (\overline{K_y \to F_1}) \mid HK_1 \ldots K_n I_1 \ldots I_r J_1 \ldots J_s)$$

$$= 1 - [\,1 - p(H{\to}F_1 \mid H\,K_1 \ldots K_n\,I_1 \ldots I_r\,J_1 \ldots J_s)]$$
$$\cdot [\,1 - p(K_1{\to}F_1 \mid H\,K_1 \ldots K_n\,I_1 \ldots I_r\,J_1 \ldots J_s)]$$
$$\vdots$$
$$\cdot [\,1 - p(K_n{\to}F_1 \mid H\,K_1 \ldots K_n\,I_1 \ldots I_r\,J_1 \ldots J_s)]$$
$$\cdot [\,1 - p(K_y{\to}F_1 \mid H\,K_1 \ldots K_n\,I_1 \ldots I_r\,J_1 \ldots J_s)].$$

Für den letzten Faktor gilt:

$$[1 - p(K_y{\to}F_1 \mid H\,K_1 \ldots K_n\,I_1 \ldots I_r\,J_1 \ldots J_s)]$$

$$= 1 - p(K_y{\to}F_1) \qquad\qquad \text{(wegen Selbständigkeit)}$$

$$= 1 - p(\,(K_y{\to}F_1)\,K_y\,)$$

$$= 1 - p(\,K_y{\to}F_1 \mid K_y)\,p(K_y)$$

$$= 1 - p(K_y{\to}F_1 \mid K_y\,\overline{H}\cdot\overline{K}_1 \ldots \overline{K}_n)\,p(K_y)$$

$$= 1 - \frac{p(F_1 \mid K_y\,\overline{H}\cdot\overline{K}_1 \ldots \overline{K}_n) - p(F_1 \mid \overline{K}_y\,\overline{H}\cdot\overline{K}_1 \ldots \overline{K}_n)}{1 - p(F_1 \mid \overline{K}_y\,\overline{H}\cdot\overline{K}_1 \ldots \overline{K}_n)}\,p(K_y)$$

$$= 1 - p(F_1 \mid K_y\,\overline{H}\cdot\overline{K}_1 \ldots \overline{K}_n)\,p(K_y)$$

$$\approx 1 \qquad\qquad (\text{ wegen } p(F_1 \mid K_y\,\overline{H}\cdot\overline{K}_1 \ldots \overline{K}_n)\,p(K_y) \ll 1).$$

Damit ergibt sich gem. Beweis des Ursachen-Faktorisierungssatzes:

$$p(F_1 \mid H\,K_1 \ldots K_n\,I_1 \ldots I_r\,J_1 \ldots J_s) = 1 - \frac{p(\overline{F}_1 \mid H\,\overline{K}_1 \ldots \overline{K}_n)}{p(\overline{F}_1 \mid \overline{H} \cdot \overline{K}_1 \ldots \overline{K}_n)}$$

$$\cdot \frac{p(\overline{F}_1 \mid \overline{H}\,K_1\,\overline{K}_2 \ldots \overline{K}_n\,I_1 \ldots I_r)}{p(\overline{F}_1 \mid \overline{H} \cdot \overline{K}_1 \ldots \overline{K}_n)}$$

$$\cdot \frac{p(\overline{F}_1 \mid \overline{H} \cdot \overline{K}_1\,K_2\,\overline{K}_3 \ldots \overline{K}_n\,J_1 \ldots J_s)}{p(\overline{F}_1 \mid \overline{H} \cdot \overline{K}_1 \ldots \overline{K}_n)}$$

$$\cdot \frac{p(\overline{F}_1 \mid \overline{H} \cdot \overline{K}_1\,\overline{K}_2\,K_3\,\overline{K}_4 \ldots \overline{K}_n)}{p(\overline{F}_1 \mid \overline{H} \cdot \overline{K}_1 \ldots \overline{K}_n)}$$

$$\vdots$$

$$\cdot \frac{p(\overline{F}_1 \mid \overline{H} \cdot \overline{K}_1 \ldots \overline{K}_{n-1}\,K_n)}{p(\overline{F}_1 \mid \overline{H} \cdot \overline{K}_1 \ldots \overline{K}_n)}$$

$$\cdot [\,1 - p(F_1 \mid K_y\,\overline{H} \cdot \overline{K}_1 \ldots \overline{K}_n)\,p(K_y)\,].$$

Somit ist

$$f = \frac{1}{\left(p(\overline{F}_1 \mid \overline{H} \cdot \overline{K}_1 \ldots \overline{K}_n)\right)^{n+1}} \cdot [\,1 - p(F_1 \mid K_y\,\overline{H} \cdot \overline{K}_1 \ldots \overline{K}_n)\,p(K_y)\,]$$

$$\approx \frac{1}{\left(p(\overline{F}_1 \mid \overline{H} \cdot \overline{K}_1 \ldots \overline{K}_n)\right)^{n+1}} \cdot 1, \quad \text{q.e.d.}$$

Ursachen-Faktorisierungssatz als Näherung

Bei verdeckten Ursachen bleibt der Ursachen-Faktorisierungssatz als Näherung gültig, wenn mit H, $K_1$, ..., $K_n$ wenigstens diejenigen Elemente erfaßt sind, die mit hoher Wahrscheinlichkeit das Ereignis $F_1$ erzeugen. Der Wert der Wahrscheinlichkeit $p(\overline{F}_1 \mid H\,K_1 \ldots K_n\,I_1 \ldots I_r\,J_1 \ldots J_s)$, ermittelt mit Hilfe des Ursachen-Faktorisierungssatzes, ist dann lediglich eine Näherung, die aber infolge der Berücksichtigung aller wesentlichen Ursachen dem wahren Wert nahe kommt. Den wahren Wert unter Berücksichtigung auch verdeckter Ursachen gibt die Glg.7.1 an. Dieser Wert ist wegen $(p(\overline{F}_1 \mid \overline{H} \cdot \overline{K}_1 \ldots \overline{K}_n))^{-1} > 1$ um den Faktor f größer als der mit dem Ursachen-Faktorisierungssatz ermittelte Wert, wobei

$$f \approx \frac{1}{(p(\overline{F}_1 \mid \overline{H} \cdot \overline{K}_1 \ldots \overline{K}_n))^{n+1}}$$

gilt und (n+1) die Anzahl der Ursachen angibt.

Kombinierter Faktorisierungssatz als Näherung

Bei Verwendung des kombinierten Faktorisierungssatzes gilt:

$$p(\overline{F}_1 \mid H\, K_1 \dots K_n\, I_1 \dots I_r\, J_1 \dots J_s) = p(\overline{F}_1 \mid H\overline{K}_1 \dots \overline{K}_n)$$

$$\cdot [\, 1 - p(F_1 \mid \overline{H}K_1\overline{K}_2 \dots \overline{K}_n) \cdot i_1 \dots i_r\,]$$

$$\cdot [\, 1 - p(F_1 \mid \overline{H} \cdot \overline{K}_1 K_2 \overline{K}_3 \dots \overline{K}_n) \cdot j_1 \dots j_s\,]$$

$$\cdot p(\overline{F}_1 \mid \overline{H} \cdot \overline{K}_1 \overline{K}_2 K_3 \overline{K}_4 \dots \overline{K}_n)$$

$$\vdots$$

$$\cdot p(\overline{F}_1 \mid \overline{H} \cdot \overline{K}_1 \dots \overline{K}_{n-1} K_n)$$

a) Fehler der ungeklammerten Faktoren:

Ist m die Anzahl der Faktoren ohne eckige Klammern, so ist der wahre Wert dieses Produkts aus m Faktoren um den Faktor $f_m$ größer als mit dem kombinierten Faktorisierungssatz angegeben, wobei

$$f_m \approx \frac{1}{(p(\overline{F}_1 \mid \overline{H} \cdot \overline{K}_1 \dots \overline{K}_n))^{m}}$$

gilt.

b) Fehler der geklammerten Faktoren

Der wahre Wert der Faktoren in eckigen Klammern ist ebenfalls größer als mit dem kombinierten Faktorisierungssatz angegeben.

Dies wird für $[1 - p(F_1 \mid \overline{H}\, K_1\overline{K}_2 \dots \overline{K}_n) \cdot i_1 \dots i_r]$ gezeigt.

1.

Für die Ermittlung des wahren Werts ist anstelle des in der Klammer verwendeten Terms $p(F_1 \mid \overline{H}\, K_1\overline{K}_2 \dots \overline{K}_n)$ jetzt $\dfrac{p(F_1 \mid \overline{H}K_1\overline{K}_2 \dots \overline{K}_n) - p(F_1 \mid \overline{H} \cdot \overline{K}_1 \dots \overline{K}_n)}{1 - p(F_1 \mid \overline{H} \cdot \overline{K}_1 \dots \overline{K}_n)}$ anzusetzen, wobei gilt:

$$p(F_1 \mid \overline{H}K_1\overline{K}_2 \dots \overline{K}_n) > \frac{p(F_1 \mid \overline{H}K_1\overline{K}_2 \dots \overline{K}_n) - p(F_1 \mid \overline{H} \cdot \overline{K}_1 \dots \overline{K}_n)}{1 - p(F_1 \mid \overline{H} \cdot \overline{K}_1 \dots \overline{K}_n)} \qquad (7.3)$$

2.

Für die Ermittlung des wahren Wert ist anstelle von

$$i_1 = \frac{p(F_1 \mid K_1 I_1 \overline{H} \cdot \overline{K}_2 \dots \overline{K}_n)}{p(F_1 \mid K_1 \overline{H} \cdot \overline{K}_2 \dots \overline{K}_n)}$$

der Quotient

$$\frac{p(F_1 \mid K_1 I_1 \overline{H} \cdot \overline{K}_2 \dots \overline{K}_n) - p(F_1 \mid \overline{H} \cdot \overline{K}_1 \dots \overline{K}_n)}{p(F_1 \mid K_1 \overline{H} \cdot \overline{K}_2 \dots \overline{K}_n) - p(F_1 \mid \overline{H} \cdot \overline{K}_1 \dots \overline{K}_n)}$$

zu verwenden, wobei gilt:

$$\frac{p(F_1|K_1 I_1 \bar{H}\cdot \bar{K}_2...\bar{K}_n)}{p(F_1|K_1\bar{H}\cdot\bar{K}_2...\bar{K}_n)} > \frac{p(F_1|K_1 I_1 \bar{H}\cdot\bar{K}_2...\bar{K}_n)\text{-}p(F_1|\bar{H}\cdot\bar{K}_1...\bar{K}_n)}{p(F_1|K_1\bar{H}\cdot\bar{K}_2...\bar{K}_n)\text{-}p(F_1|\bar{H}\cdot\bar{K}_1...\bar{K}_n)}. \tag{7.4}$$

Es müssen somit in $[1 - p(F_1|\bar{H}K_1\bar{K}_2...\bar{K}_n)\cdot i_1...i_r]$ anstelle von $i_1, ..., i_r$, und anstelle von $p(F_1|\bar{H}K_1\bar{K}_2...\bar{K}_n)$ kleinere Größen angesetzt werden, so daß insgesamt $[1 - p(F_1|\bar{H}K_1\bar{K}_2...\bar{K}_n)\cdot i_1...i_r]$ größer wird.
Ende.

**Patentansprüche**

1. Verfahren zur Ermittlung von Wahrscheinlichkeiten pathophysiologischer Zustände als Zwischenergebnisse der Diagnosefindung, dadurch gekennzeichnet, daß man unter Verwendung der Bezeichnungen des Anspruchs 4 und unter Verwendung geeigneter Voraussetzungen für ein beliebiges Ereignis H mit der unbekannten Zutreffenswahrscheinlichkeit $0< p(H \mid H')< 1$ die Beziehung

$$p(H|U\ F\ K\ I\ J)= \frac{1}{1+\dfrac{p(F|KJ\bar{H})p(\bar{H}|UI)}{p(F|KJH)p(H|UI)}}$$

anwendet, und daß man dann für $F := (F_1\ F_2\ F_3...F_r)$ eine r-fache Anwendung des L-Satzes auf die rechte Seite der Gleichung

$$p(F|K\ J\ H) = p(F_1|F_2 F_3...\ F_r\ K\ J\ H)p(F_2|F_3\ ...\ F_r\ K\ J\ H)\dot{\ }.....\dot{\ }p(F_r|K\ J\ H)$$

ausführt, äquivalent einer linearen Interpolation von $p(F|K\ J\ H)$.

2. Verfahren gemäß Anspruch 1 oder Verfahren unter Verwendung einer vergleichbaren Umformung von $p(H|U\ F\ K\ I\ J)$, dadurch gekennzeichnet, daß man für die in einer solchen Umformung erscheinenden Wahrscheinlichkeiten der Form z.B. $p(F|K\ J\ H)$ und $p(H|U\ I)$, in einer verallgemeinerten Schreibweise zusammenfassend also für Wahrscheinlichkeiten der Form $p(H|E_1...E_h E'_{h+1}...E'_k)$, die Beziehung

$$p(H \mid E_1...E_h E'_{h+1}...E'_k) = \sum_{q_{h+1},...,q_k=0,1} p(H \mid E_1...E_h E_{h+1}^{(q_{h+1})}...E_k^{(q_k)})\prod_{i=h+1}^{k} p(E_i^{(q_i)} \mid E'_i)\ ,$$

$$E_i^{(0)} = \overline{E_i}\ \text{und}\ E_i^{(1)} = E_i,\ \text{i} := \text{h}+1, ..., \text{k},$$

genau dann einsetzt, wenn gilt:
$\{E_{h+1}, ..., E_k\}$ sind stochastisch unabhängig unter der Bedingung des Ereignisverbundes $(E_1 ... E_h\ W(E_{h+1})^* ...\ W(E_k)^*)$, wobei der Verbund $W(E_i)^*$ für alle i definiert ist als $W(E_i)^* := (\ W(E_i)$ abzgl. $\{H\}\cup\{E_1,..., E_h\})$.

3. Verfahren gemäß Anspruch 1 oder 2, dadurch gekennzeichnet, daß man im Falle einer Nicht-Anwendung der in Anspruch 1 oder 2 angegebenen linearen Interpolationen, oder im Falle von nicht-linearen Interpolationen zusätzlich zu den Interpolationen des Anspruchs 1 oder 2, die Beziehung

$$p(H \mid E_1...E_h E_{h+1}^{'}...E_k^{'}) =$$

$$\frac{\sum_{q_{h+1},...,q_k=0,1} p(HE_1...E_h E_{h+1}^{(q_{h+1})}...E_k^{(q_k)}) \prod_{i=h+1}^{k} \frac{p(E_i^{(q_i)} \mid E_i^{'})}{p(E_i^{(q_i)} \mid E_1...E_h E_{h+1}^{'}...E_{i-1}^{'} E_{i+1}^{'}...E_k^{'})}}{\sum_{q_{h+1},...,q_k=0,1} p(E_1...E_h E_{h+1}^{(q_{h+1})}...E_k^{(q_k)}) \prod_{i=h+1}^{k} \frac{p(E_i^{(q_i)} \mid E_i^{'})}{p(E_i^{(q_i)} \mid E_1...E_h E_{h+1}^{'}...E_{i-1}^{'} E_{i+1}^{'}...E_k^{'})}},$$

$$E_i^{(0)} = \overline{E_i} \ \text{und} \ E_i^{(1)} = E_i, \ \text{i} = \text{h} + 1, ..., \text{k}$$

einsetzt, daß man für sämtliche nicht-linearen Interpolationen genau diese Beziehung anwendet, und daß dafür der Term $p(E_i^{(q_i)}|E_1...E_h E'_{h+1}...E'_{i-1}E_{i+1}...E'_k)$ in der Formel stets verwendet wird.

**4.** Verfahren gemäß Anspruch 1 oder 2 oder 3, dadurch gekennzeichnet, daß man Ereignisse nicht uneingeschränkt oder mit beliebigen Voraussetzungen zuläßt, sondern daß man für kausal geordnete Ereignisse bei Verwendung der Bezeichnungen

| | |
|---|---|
| U | Verbund der Ursachen von H, |
| F | Verbund der Folgen von H, |
| K | Verbund der Ereignisse, die mit H eine gemeinsame Folge besitzen, |
| I | Verbund der Inhibitoren von Kausalverbindungen, die zu H führen, |
| J | Verbund der Inhibitoren von Kausalverbindungen, die zu Folge-Ereignissen von H führen, |
| URS(H) | Menge der Ursachen von H, |
| FOL(H) | Menge der Folgen von H, |

als Voraussetzungen fordert, daß

- die Ereignisse aus F, I, J mit der Wahrscheinlichkeit Null oder Eins vorliegen,
- (H K), im Bedarfsfall auch U, sämtliche, diesen Verbunden zuzuordnenden Ereignisse enthalten,
- die Ereignisse in (H K), im Bedarfsfall auch die Ereignisse in U, selbständige Ursachen sind,
- die Ereignisse in

$$\bigcup_{F_i \in F} (URS(F_i^{'}) \cup INH(F_i^{'})) \ ,$$

im Bedarfsfall auch die Ereignisse in URS(H) $\cup$ INH(H), stochastische Unabhängigkeit aufweisen, und daß
- p(U F K I J|H) = p(U I|H) p(F K J|H) erfüllt ist,

wobei die 1. Strichzählung wahlweise besteht, wobei eine (Nahezu-) Äquivalenz zwischen der 3. und der 4. Strichzählung genutzt werden kann, und wobei der angeführte Bedarfsfall genau dann eintritt, wenn URS(H) '-gekennzeichnete Elemente enthält, deren Berechnungen die analogen Voraussetzungen erfordern, wie sie für die Berechnung von p(H|H') erforderlich sind.

**5.** Verfahren gemäß Anspruch 1 oder 2 oder 3, dadurch gekennzeichnet, daß man anstelle der in Anspruch 4 angegebenen Voraussetzungen fordert, daß

- die Ereignisse aus U, F, I, J mit der Wahrscheinlichkeit Null oder Eins vorliegen,
- (H K) sämtliche, diesem Verbund zuzuordnenden Ereignisse enthält,
- die Ereignisse in (H K) Selbständigkeitseigenschaft aufweisen,
- die Ereignisse in

$$\bigcup_{F_i \in F} (URS(F_i) \cup INH(F_i))$$

stochastisch unabhängig sind,

- $p(U\ F\ K\ I\ J|H) = p(U\ I|H)\ p(F\ K\ J|H)$ erfüllt ist,

wobei die 1. Strichzählung wahlweise besteht, und wobei eine (Nahezu-) Äquivalenz zwischen der 3. und der 4. Strichzählung genutzt werden kann.

6.   Verfahren gemäß Anspruch 1 oder 2 oder 3, jeweils mit Anspruch 4 oder 5, dadurch gekennzeichnet, daß man die in Anspruch 1 genannten Wahrscheinlichkeiten der Form $p(F|K\ J\ H)$ und $p(H|UI)$, oder die gemäß Anspruch 2 mit einer anderen Umformung von $p(H|U\ F\ K\ 1\ J)$ erreichten bedingten Wahrscheinlichkeiten, nach erfolgter Interpolation nicht beläßt, sondern z.B. die Beziehung

$$p(F \mid K_1...K_n JH) = \prod_i p(F_i \mid K_1...K_n JH)$$

verwendet, wobei exemplarisch $K := (\ K_1\ ...\ K_n)$ gesetzt ist, daß man dann für alle i die Gleichung

$$p(F_i \mid K_1 ... K_n\ J\ H) = \ 1\text{-}\ (1\text{-}\ p(H.F_i \mid K_1...K_n\ J\ H)\ )\cdot \prod_{j=1}^{n}(1 - p(K_j.F_i \mid K_1...K_n.JH))$$

nutzt, und daß man schließlich auf die darin enthaltenen Wahrscheinlichkeiten der Form $p(H.F_i|K_1...K_n\ J\ H)$ bzw. $p(K_j.F_i|K_1...K_n\ J\ H)$ den A→L-Satz oder das A→L-Korollar 1 anwendet.

7.   Verfahren gemäß Anspruch 1 bis 6, dadurch gekennzeichnet, daß man zur Bestimmung der Zutreffenswahrscheinlichkeit von H insbesondere auch kausal mit dem Ereignis H verknüpfte Ereignisse $X_1, ... , X_m$ mit den unbekannten Zutreffenswahrscheinlichkeiten $p(X_1|X_1'), ... , p(X_m|X_m')$ berücksichtigt, indem man

   1.
   jeweils eine kausale Strukturierung zugrundelegt, die den in Anspruch 4 oder in Anspruch 5 geforderten Voraussetzungen genügt, daß man
   2.
   für ein beliebiges, in der Kausalstruktur enthaltenes Ereignis $X_1$, dessen Zutreffenswahrscheinlichkeit $p(X_1|X_1')$ unbekannt ist, eine sogenannte Wertungsgleichung $p(X_1|X_1') = p(X_1|W(X_1))$ aufstellt, wobei der Bedingungsverbund $W(X_1)$ unmittelbar mit $X_1$ verknüpfte und gegenüber $X_1$ stochastisch abhängige Ereignisse enthält, die negiert, unnegiert oder '-gekennzeichnet auftreten, daß man dies für alle m Ereignisse $X_1, ... , X_m$ mit unbekannter Zutreffenswahrscheinlichkeit ausführt, und daß man
   3.
   die Wertungsgleichungen in die Form gemäß Anspruch 1 oder in eine vergleichbare Form bringt, daß man die erhaltenen bedingten Wahrscheinlichkeiten nach Möglichkeit linear gemäß Anspruch 1 oder 2, nötigenfalls auch nicht-linear gemäß Anspruch 3 interpoliert, daß man
   4.
   eine Verkleinerung der bedingten Wahrscheinlichkeiten ausführt, zuächst bezüglich der vor dem Bedingungsstrich stehenden Ereignisse, unter Nutzung der Vollzähligkeit der beteiligten Ursachen und der Unabhängigkeit aller, auch der unbekannten Inhibitoren, daß man
   5.
   die bedingten Wahrscheinlichkeiten weiter verkleinert, jetzt bezüglich der Ereignisse nach dem Bedingungsstrich, unter Nutzung der für die beteiligten Ursachen eingeforderten Selbständigkeitseigenschaft, wobei man den Anspruch 6 verwendet, und daß man
   6.
   das solcherart aufgestellte Gleichungssystem von m Gleichungen in den m Unbekannten $p(X_1|X_1'), ... , p(X_m|X_m')$ iterativ mit Hilfe des nicht-linearen Einzel- oder Gesamtschrittverfahrens, oder mit einem beliebigen anderen iterativen Verfahren löst.

8.   Verfahren gemäß Anspruch 6, dadurch gekennzeichnet, daß man die in

$$p(F \mid K_1 ... K_n JH) = \prod_i p(F_i \mid K_1 ... K_n JH)$$

erscheinenden Wahrscheinlichkeiten, z.B. $p(F_1 \mid K_1 ... K_n I_1 ... I_r J_1 ... J_s H)$, mit Hilfe der aus dem A→L-Satz, dem A→L-Korollar 1 und den getroffenen Voraussetzungen unmittelbar herleitbaren sogenannten Ursachen-Faktorisierung

$$p(\overline{F}_1 \mid K_1 ... K_n I_1 ... I_r J_1 ... J_s H) = p(\overline{F}_1 \mid H\overline{K}_1...\overline{K}_n)$$
$$\cdot p(\overline{F}_1 \mid \overline{H}K_1\overline{K}_2...\overline{K}_n I_1...I_r)$$
$$\cdot p(\overline{F}_1 \mid \overline{H} \cdot \overline{K}_1 K_2 \overline{K}_3...\overline{K}_n J_1...J_s)$$
$$\cdot p(\overline{F}_1 \mid \overline{H} \cdot \overline{K}_1 \overline{K}_2 K_3 \overline{K}_4...\overline{K}_n)$$
$$\vdots$$
$$\cdot p(\overline{F}_1 \mid \overline{H} \cdot \overline{K}_1...\overline{K}_{n-1} K_n)$$

weiter bearbeitet, wobei exemplarisch

$I_1, ... , I_r$ die Inhibitoren von $K_1 \rightarrow F_1$ und
$J_1, ... , J_s$ die Inhibitoren von $K_2 \rightarrow F_1$

darstellen, und wobei insbesondere vorausgesetzt ist, daß $\{H, K_1, ..., K_n\}$ sämtliche Ursachen von $F_1$ enthält, und daß alle Ursachen selbständige Ursachen von $F_1$ sind

9. Verfahren gemäß Anspruch 8, dadurch gekennzeichnet, daß man anstelle der Ursachen-Faktorisierung die sogenannte kombinierte Faktorisierung

$$p(\overline{F}_1 \mid K_1 ... K_n I_1 ... I_r J_1 ... J_s H) = p(\overline{F}_1 \mid H\overline{K}_1...\overline{K}_n)$$
$$\cdot [ 1 - p(F_1 \mid \overline{H}K_1\overline{K}_2...\overline{K}_n) \cdot i_1...i_r ]$$
$$\cdot [ 1 - p(F_1 \mid \overline{H} \cdot \overline{K}_1 K_2 \overline{K}_3...\overline{K}_n) \cdot j_1...j_s ]$$
$$\cdot p(F_1 \mid \overline{H} \cdot \overline{K}_1 \overline{K}_2 K_3 \overline{K}_4...\overline{K}_n)$$
$$\vdots$$
$$\cdot p(\overline{F}_1 \mid \overline{H} \cdot \overline{K}_1...\overline{K}_{n-1} K_n) .$$

nutzt, die ebenso wie die Ursachen-Faktorisierung aus den Gegebenheiten des Anspruchs 6 abgeleitet wird, wobei exemplarisch

$I_1, ... , I_r$ die Inhibitoren von $K_1 \rightarrow F_1$ und
$J_1, ... , J_s$ die Inhibitoren von $K_2 \rightarrow F_1$

darstellen, wobei die Zuweisungen

$$i_1 := \frac{p(F_1 \mid K_1 I_1 \overline{H} \cdot \overline{K}_2...\overline{K}_n)}{p(F_1 \mid K_1 \overline{H} \cdot \overline{K}_2...\overline{K}_n)}, \; ..., \; i_r := \frac{p(F_1 \mid K_1 I_r \overline{H} \cdot \overline{K}_2...\overline{K}_n)}{p(F_1 \mid K_1 \overline{H} \cdot \overline{K}_2...\overline{K}_n)},$$

$$J_1 := \frac{p(F_1|\bar{H}\cdot\bar{K}_1 K_2 \bar{K}_3 ... \bar{K}_n J_1)}{p(F_1|\bar{H}\cdot\bar{K}_1 K_2 \bar{K}_3 ... \bar{K}_n)}, ..... , j_s := \frac{p(F_1|\bar{H}\cdot\bar{K}_1 K_2 \bar{K}_3 ... \bar{K}_n J_s)}{p(F_1|\bar{H}\cdot\bar{K}_1 K_2 \bar{K}_3 ... \bar{K}_n)},$$

gelten, und wobei insbesondere vorausgesetzt ist, daß die Menge {H, $K_1$, ..., $K_n$} sämtliche Ursachen des Ereignisses $F_1$ enthält, daß alle Ursachen selbständige Ursachen von $F_1$ sind, und daß die Inhibitoren selbständige Ursachen der betroffenen negierten Erzeugungsvorgänge sind.

10. Verfahren gemäß Anspruch 8 oder Anspruch 9, dadurch gekennzeichnet, daß man bei der Herleitung der Faktorisierungssätze anhand der Gegebenheiten des Anspruchs 6 den Term $p(\bar{F}_1|\bar{H}\cdot\bar{K}_1...\bar{K}_n)$ nicht gleich Eins setzt, sondern daß man bei der Existenz verdeckter und damit nicht berücksichtigter Ursachen eine Zuweisung $p(\bar{F}_1|\bar{H}\cdot\bar{K}_1...\bar{K}_n) < 1$ verwendet, so daß im Fall einer Anwendung der Ursachen-Faktorisierung, und auch näherungsweise im Fall einer Anwendung der kombinierten Faktorisierung, der gemäß Anspruch 8 oder 9 zum Beispiel für $p(\bar{F}_1|H\ K_1\ ...\ K_n\ I_1\ ...\ I_r\ J_1\ J_s)$ ermittelte Wert mit f zu multiplizieren ist, wobei

$$f \approx \frac{1}{(p(\bar{F}_1|\bar{H}\cdot\bar{K}_1...\bar{K}_n))^{n+1}},$$

n+ 1 := Anzahl der Ursachen.

Europäisches
Patentamt

**EUROPÄISCHER RECHERCHENBERICHT**

Nummer der Anmeldung

EP 99 10 5884

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG |
|---|---|---|---|
| X | EP 0 504 457 A (LIEBEL FRANZ PETER DR) 23. September 1992 (1992-09-23) | 1-4 | G06F19/00 G06F17/18 |
| A | * Seite 3, Zeile 1 - Seite 10, Zeile 25 * * Seite 22, Zeile 20 - Seite 24, Zeile 10 * | 5-10 | |
| A | EP 0 281 682 A (LIEBEL FRANZ PETER DR) 14. September 1988 (1988-09-14) * Seite 2, Zeile 1 - Zeile 24 * | 1-10 | |
| A | EP 0 399 082 A (LIEBEL FRANZ PETER DR) 28. November 1990 (1990-11-28) * Seite 2, Zeile 3 - Zeile 17 * | 1-10 | |
| A | EP 0 400 354 A (LIEBEL FRANZ PETER DR) 5. Dezember 1990 (1990-12-05) * Zusammenfassung * | 1-10 | |

RECHERCHIERTE SACHGEBIETE

G06F

Der vorliegende Recherchenbericht wurde für alle Patentansprüche erstellt

| Recherchenort | Abschlußdatum der Recherche | Prüfer |
|---|---|---|
| DEN HAAG | 19. Juli 1999 | Schenkels, P |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

&  : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 99 10 5884

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-07-1999

| Im Recherchenbericht angeführtes Patentdokument | | Datum der Veröffentlichung | Mitglied(er) der Patentfamilie | | Datum der Veröffentlichung |
|---|---|---|---|---|---|
| EP 0504457 | A | 23-09-1992 | KEINE | | |
| EP 0281682 | A | 14-09-1988 | DE | 3705964 A | 08-09-1988 |
| | | | DE | 3710559 A | 13-10-1988 |
| | | | DE | 3713692 A | 17-11-1988 |
| EP 0399082 | A | 28-11-1990 | EP | 0400354 A | 05-12-1990 |
| EP 0400354 | A | 05-12-1990 | EP | 0399082 A | 28-11-1990 |

EPO FORM P0461

Für nähere Einzelheiten zu diesem Anhang : siehe Amtsblatt des Europäischen Patentamts, Nr. 12/82